# EUROPÄISCHE PATENTANMELDUNG

(11) **EP 3 354 292 A1**
(43) Veröffentlichungstag der Anmeldung: **01.08.2018**
(21) Anmeldenummer: 17153810.1
(22) Anmeldetag: 30.01.2017
(51) Int. Cl.: A61L 31/12

(54) **HERSTELLUNG VON RESORBIERBAREN POLYMERROHREN AUS MEHRKOMPONENTENFÄDEN**

(71) Anmelder: MeKo Laserstrahl-Materialbearbeitungen e.K., 31157 Sarstedt (DE)
(72) Erfinder: Die Erfindernennung liegt noch nicht vor
(74) Vertreter: Arth, Hans-Lothar

(57) **Zusammenfassung**

Die vorliegende Erfindung betrifft Polymerrohre und bevorzugt bioresorbierbare Polymerrohre hergestellt aus mindestens einem Mehrkomponentenfaden, wobei der Mehrkomponentenfaden einen Durchmesser von ≤ 200 µm aufweist sowie Verfahren zur Herstellung dieser Polymerrohre, welche insbesondere für die Herstellung von Stents geeignet sind. Der aufgewickelte mindestens eine Mehrkomponentenfaden kann zu einem porenfreien massiven Polymerrohr vereinigt werden ohne Verlust der Molekülorientierung.

## Beschreibung

Die vorliegende Erfindung betrifft Polymerrohre, bevorzugt aus bioresorbierbaren Polymeren, hergestellt aus mindestens einem Mehrkomponentenfaden, wobei der mindestens eine Mehrkomponentenfaden einen Durchmesser von ≤ 200 µm aufweist und mindestens eine Polymerfaser enthält, die entlang der Fadenlängsachse des aufgewickelten mindestens einen Mehrkomponentenfadens ausgerichtet ist, sowie ein Verfahren zur Herstellung dieser Polymerrohre, welche insbesondere für die Herstellung von Stents geeignet sind.

Die Implantation von Gefäßstützen wie beispielsweise Stents ist heutzutage ein gängiger interventioneller Eingriff zur Behandlung von Stenosen. Sie werden üblicherweise aus Metalllegierungen, wie nichtrostendem Edelstahl, Kobalt-Chromlegierungen oder Nickeltitan (z. B. Nitinol) hergestellt. Derartige Metallstents sind in großer Zahl bekannt und haben sich in der Praxis bewährt. Zum einen sollen derartige Metallstents aufgrund ihrer Metallstruktur und radialen Festigkeit gewährleisten, dass die Blutgefäße nach einer Aufdehnung und Stent-Implantation offen bleiben und der Blutfluss durch die Gefäße dauerhaft sichergestellt ist. Zum anderen dienen Stents in der Krebsbehandlung dazu, durch bösartige Tumoren verursachte Verengungen von Atemwegen (Luftröhre), Gallenwegen oder der Speiseröhre zu verhindern oder nach einer Aufdehnung offenzuhalten.

Stents werden derzeit in zwei Grundtypen eingeteilt: dauerhafte (biostabile) und resorbierbare (degradierbare) Stents. Dauerhafte Stents sind so ausgestaltet, dass sie im Gefäß für einen unbestimmten Zeitraum verbleiben können. Resorbierbare Stents werden hingegen über einen vorbestimmten Zeitraum hinweg im Gefäß abgebaut.

Neuere Untersuchungen haben gezeigt, dass Gefäßstenosen nicht permanent durch eine Endoprothese, insbesondere in Form eines Stents, gestützt werden müssen. Es ist vollkommen ausreichend, das Blutgefäß für einen begrenzten Zeitraum zu stützen, bis das traumatisierte Gewebe des Gefäßes verheilt und sich die glatten Gefäßmuskelzellen regeneriert haben. Sie übernehmen dann wieder die Aufgabe, das Blutgefäß offen zu halten, weshalb der Stent nicht länger erforderlich ist und nicht im Gefäßlumen verbleiben muss. Diese zeitlich begrenzte Funktion der Gefäßstützung wird durch sogenannte resorbierbare Stents erfüllt, die im Körper abgebaut werden.

Resorbierbare Stents haben den Vorteil, dass das körperfremde Material nicht dauerhaft im Gefäß verbleibt und die Gefahr einer negativen Gefäßreaktion wie einer Restenose somit zeitlich begrenzt wird. Bei Kindern besteht für resorbierbare Stents zusätzlich der Vorteil, dass die Gefäße in ihrem Wachstum nicht durch eine permanente Gefäßstütze gehemmt werden. Sollte sich nach der Resorption des Stents ein erneuter Therapiebedarf an der gleichen Stelle ergeben, stehen dem Patienten wieder alle Therapieoptionen offen. Resorbierte Stents gelten im Gegensatz zu biostabilen Stents nicht als Kontraindikation für herzchirurgische Bypass-Operationen. Als besonders kritisch gilt darüber hinaus die sogenannte In-Stent-Restenose (ISR), die ebenfalls mit bioresorbierbaren Stents therapiert werden kann.

Derzeit sind zwei grundsätzlich unterschiedliche Materialien für die bioresorbierbaren Stents im Einsatz bzw. in der klinischen Untersuchung:
- Biopolymere, d.h. resorbierbare Polymere, insbesondere Polylactide
- Metalle, insbesondere Magnesiumlegierungen

Bezüglich der bioresorbierbaren Metall-Stents hat bislang nur die Firma Biotronik klinische Studien mit einer bioresorbierbaren Magnesium-Legierung durchgeführt und hat im Juni 2016 ein CE-Zeichen für den bioresorbierbaren Magnesiumstent Magmaris erhalten. Dabei kann Biotronik bislang nur die Sicherheit des Systems belegen, detaillierte Vergleiche mit anderen Systemen stehen noch aus. Die Firma Lifetech (China) hat hingegen erste Tierversuche mit einem eisenbasierten, resorbierbaren Stent durchgeführt. Von anderen Firmen sind keine klinischen Studien oder präklinischen Studien am Tiermodel bekannt. Obgleich grundsätzlich das angestrebte Ziel einer Resorption des implantierten Stents erreicht wird, besteht bei Magnesiumlegierungen bislang das Problem eines zeitlich nicht definierten Abbaus des Stents. Je nach Legierung ist der Materialabbau starken Schwankungen unterworfen, schwer kontrollierbar und im Allgemeinen zu schnell, um eine Übernahme der Stützfunktion bis zur Regeneration des Gefäßabschnitts zu gewährleisten. Durch eine unkontrollierte Degradation können sich Bruchstücke des Stents ablösen und Thrombosen verursachen.

Die meisten Entwicklungen resorbierbarer Stents konzentrieren sich aber auf Biopolymere und hierbei auf Polylactide, auch Polymilchsäuren genannt (kurz PLA, vom englischen Wort *polylactic acid*)*.* Sie sind technische Biopolymere, gehören zu den Polyestern und sind aus vielen, chemisch aneinander gebundenen Milchsäuremolekülen aufgebaut.

Von den Polylactiden werden insbesondere das Poly-L-lactid (PLLA) und das Polylactid-co-Glycolid (PLGA) für resorbierbare Stents verwendet.

Als Erste hat Kyoto Medical im Nov. 2007 eine CE-Zulassung für einen unbeschichteten PLLA Stent Igaki-Tamai^{®} erhalten. Später gelang Abbott für den Absorb^{®} die CE-Zulassung; ein PLLA Stent mit Everolimus als Wirkstoff in einer Poly-D,L-Lactid (PDLLA) Beschichtung. Im Jahr 2013 hat Elixir die Zulassung für seinen PLLA basierten Stent Desolve^{®} mit einer Novolimus enthaltenden Beschichtung erhalten.

Polylactide sind problemlos verfügbar. Es gibt mehrere Hersteller mit langjährigen Erfahrungen. Das Material gilt als körperverträglich. Aus der Chirurgie ist bioresorbierbares Polylactid-Nahtmaterial seit Jahren bekannt und im Einsatz. Folglich existieren keine Einwände Polylactide als Implantatmaterial zu verwenden und es müssen keine umfangreichen Biokompatibilitäts-Untersuchungen durchgeführt werden. Die Resorbierbarkeit lässt sich durch Wahl der geeigneten Polylactide bzw. Materialmischungen (Co-polymere) genau einstellen. Derzeit strebt man eine völlige Degradation, d.h. einen völligen Abbau der Stents bzw. Stentstreben innerhalb von 18 Monaten (1,5 Jahre) nach Implantation an.

Die Polylactide PLLA und PLGA weisen als Werkstoff für Stents, das heißt in Form eines Rohrs, im Allgemeinen eine hohe Bruchdehnung auf. Bruchdehnungen bis zu 100 % für ein handelsübliches PLLA Rohr (ø 1,8 x 0,15 mm) sind problemlos erreichbar; allerdings mit dem Nachteil, dass die Festigkeit dieser Rohre nur 50 - 60 MPa beträgt. Festigkeitssteigernde Maßnahmen führen hingegen zu einer Verringerung der Bruchdehnung und sind nur begrenzt durchführbar. Den Vorteilen der Verwendung von Polylactiden steht somit auch eine Reihe von Nachteilen gegenüber.

Als größter Nachteil der Polylactide ist die begrenzte Festigkeit des Materials zu nennen, weshalb Verfahren zur Festigkeitssteigerung zum Einsatz kommen. PLLA Rohre zeigen bei Zugversuchen eine Festigkeit von unter 60 MPa (typischerweise 50 MPa). Aufgrund der geringen Festigkeit der Polylactide sind hohe Wandstärken bei den Stentrohlingen (Polymerrohre) respektive den Stentstreben notwendig, damit die Stents eine ausreichende radiale Kraft zur Gefäßstützung aufweisen.

Große Anstrengungen werden unternommen, um die Festigkeit der Polymere bzw. der Rohre zu steigern. Dennoch betragen heutzutage die Rohr- bzw.

Stegwandstärken mindestens 150 µm, um eine ausreichende radiale Kraft zu gewährleisten. Mit diesen Wandstärken sind kleine Gefäßdurchmesser nicht erreichbar. Diese massiven Stege stehen im Widerspruch zu dem Trend, die derzeit nicht resorbierbaren Stents mit immer dünneren Stegen herzustellen mit Stegquerschnitten von teilweise nur noch 60 µm und darunter. Man erwartet von diesen filigranen Stents bessere Heilungschancen für das Gefäß, da der Blutfluss weniger stark beeinträchtigt wird und weniger Gefäßwandfläche abgedeckt wird.

Die festigkeitssteigernden Maßnahmen führen wiederum zu einer geringeren Bruchdehnung der Polymere, weshalb bereits geringe Überdehnungen der Polylactid-Stents zu Brüchen einzelner Stege führen. Trotz der hohen Stegquerschnitte ist die Festigkeit der Stentstreben oder auch Stege begrenzt, was in Kombination mit der geringen Bruchdehnung (der Bruchempfindlichkeit) zu einer kostenaufwändigen und langwierigen Prozedur bei Stentimplantationen führt.

So wird empfohlen, die PLLA Stents nicht direkt zu implantieren ("direct stenting"), sondern mit Hilfe einer Ballondilatation das stenotisierte Gefäß zuvor aufzudehnen ("pre-dilatation"). Teilweise wird noch eine Atherektomie durchgeführt, d.h. mittels minimal-invasiver Methoden werden die harten Ablagerungen in den Arterien entfernt. Danach soll in schwierigen Fällen der Gefäßdurchmesser vorzugsweise mittels der IVUS oder OCT Methode präzise vermessen werden, um die richtige Stentgröße auszuwählen und damit eine Über- oder Unterdehnung des Polylactid-Stents zu vermeiden. Schließlich wird der Polylactid-Stent dann in das bereits geöffnete und vorbereitete Gefäß eingesetzt. Eine Post-Dilatation des implantierten Stents mit einem non-compliant Ballonkatheter wird zusätzlich empfohlen.

Als weiterer Nachteil der Polylactid-Stents sei hier die begrenzte Lagerfähigkeit aufgrund der Empfindlichkeit des Materials gegenüber Temperatur und Feuchtigkeit genannt. Unerwünschte Zersetzungsprozesse können frühzeitig vor der Implantation beginnen.

Polymere allgemein neigen zudem zum Kriechen, insbesondere gedehnte Strukturen aus den Polymeren zeigen dieses Verhalten. Auf den Ballonkatheter gecrimpte Stents dehnen sich ganz langsam und allmählich wieder etwas auf, wodurch der Stent keinen festen Sitz mehr auf dem Ballonkatheter hat. Im Gefäß aufgedehnte Stents ziehen sich wieder leicht zusammen und verringern damit den Gefäßquerschnitt respektive den Blutfluss.

Rohre für die Herstellung von Gefäßimplantaten bzw. Stents weisen kleine Durchmesser von typischerweise unter 3,0 mm auf. Bestehen diese Rohre aus Biopolymeren, werden sie bevorzugt durch Extrusion hergestellt. Bei einer Herstellung durch Extrusion (Strangpressen) wird ein hochreines trockenes Polylactid-Granulat in kleinen Extrudern zu einer dickflüssigen Masse erschmolzen und durch einen Schneckentrieb kontinuierlich unter hohem Druck aus einer Düse als Rohr ausgepresst.

Ein weiteres Herstellungsverfahren ist das Spritzgießen (Spritzguss). Dazu wird mit einer Spritzgießmaschine der jeweilige Werkstoff in einer Spritzeinheit plastifiziert und in ein Spritzgießwerkzeug eingespritzt. Der Hohlraum, die Kavität, des Werkzeugs bestimmt die Form und die Oberflächenstruktur des fertigen Teils. Alternativ können Polymerrohre auch aus Hohlkörpern gezogen werden: Rohrziehprozess.

Den Herstellungsverfahren Extrusion und Spritzgießen ist gemein, dass die langkettigen Polylactid-Molekülketten weitgehend ungerichtet sind bzw. leicht in Pressrichtung (axiale Rohrrichtung) vororientiert sind und die Rohre in einem weitgehend spannungsfreien Zustand vorliegen. Die Zugfestigkeiten der Rohre sind mit 50 bis 60 MPa gering. Ausreichende radiale Festigkeiten der Stents lassen sich mit diesen Polylactid-Rohren nur durch hohe Wandstärken bzw. große Querschnitte der Stentstreben erreichen. Ein Vorteil der mittels Extrusion oder Spritzgießen hergestellten Polymerrohre sowie der daraus gewonnenen Stents ist, dass die Polymerrohre und Stents nicht porös sind, d.h. die Polymerrohre und die Stentstreben weisen keine Poren oder Zwischenräume auf, wie es bei Polymerrohren aus aufgewickelten Fäden der Fall ist und haben daher den Vorteil, sich gleichmäßig unter physiologischen Bedingungen abzubauen.

Hier weisen Polymerrohre, welche durch Aufwickeln von Polymerfäden mittels Lösungsspinnen oder Schmelzspinnen erhalten werden, den Nachteil auf, dass gewebeartige oder filzartige Strukturen mit Poren oder Zwischenräumen zwischen den aufgewickelten Fäden entstehen, so dass kein gleichmäßiger biologischer Abbau unter physiologischen Bedingungen stattfindet und Bruchstücke derartiger Rohre vor allem im vaskulären Bereich Komplikationen bis hin zum Herzinfarkt verursachen können. Vorteil dieser durch Aufwicklung von Fäden erhaltenen Polymerrohre ist hingegen die Ausrichtung der Molekülketten entlang der Fadenlängsachse, wodurch höhere Zugfestigkeiten erhalten werden, so dass sich derartige Polymerrohre nur für große Körperöffnungen eignen und aus nicht bioresorbierbaren Polymeren bestehen wie beispielsweise Speiseröhrenstents in Form von einem Rohr aus aufgewickelten Fäden mit Poren, welche eine Größe haben, dass eine Krebszelle diese nicht mehr passieren kann. Ein derartiger Speiseröhrenstent ist beispielsweise in der US-Patentanmeldung US 2013103139 A1 offenbart. Dieser Stent weist zwar eine höhere Zugfestigkeit auf aber keine Biegesteifigkeit und damit keine Radialkraft wie sie für Stents und Gefäßstützen und insbesondere Koronarstents zwingend erforderlich ist, damit der gewünschte Gefäßdurchmesser durch den Stent offengehalten werden kann.

Die internationale Offenlegungsschrift WO 99/17817 A1 offenbart ein poröses Gewebe aus ungeordneten Filamenten sowie eine Vorrichtung und ein Verfahren zur Herstellung dieser Gewebe. Figur 1 von WO 99/17817 A1 zeigt die Vorrichtung und Figur 2 das Gewebe aus einem Gewirr ungeordneter Fäden. Weder ein porenfreies Polymerrohr noch eine gerichtete oder geordnete Ausrichtung der Polymerfäden werden in WO 99/17817 A1 offenbart. WO 99/17817 A1 beschreibt auch kein porenfreies Verschmelzen der Polymerfäden sondern ein nicht-porenfreies Verschmelzen der Polymerfäden unterhalb der Schmelztemperatur.

Die US-Offenlegungsschrift US 2010/0070020 A1 offenbart einen aus Polyurethanfasern elektrogesponnenen Grundkörper, der dann mit einem Stützgewebe ummantelt und nochmals mit einer äußeren Schicht umrundet wird. Figur 2A von US 2010/0070020 A1 zeigt eine elektronenmikroskopische Aufnahme elektrogesponnener willkürlich ausgerichteter Polymerfasern. Weder ein porenfreies Polymerrohr noch eine gerichtete oder geordnete Ausrichtung der Polymerfäden werden in US 2010/0070020 A1 offenbart.

Die internationale Offenlegungsschrift WO 2015/091357 A1 offenbart ein Polymerrohr aus gerichtet aufgewickelten Polymerfäden, welche danach porenfrei verschmolzen wurden, so dass sich ein massives porenfreies Polymerrohr wie bei einem Extrusions- oder Spritzgießverfahren ergibt. Jedoch konnte durch das Verschmelzen die Ausrichtung der Polymerketten oder Molekülketten in den Polymerfäden nicht vollständig erhalten werden, wodurch nur gering gesteigerte Zugfestigkeiten erhalten werden und eine geringe Rückstellkraft in Richtung Nominaldurchmesser des Polymerrohres erhalten werden konnte.

Durch alternierendes oder gemeinsames Aufwickeln zweier unterschiedlicher Polymerfäden bestehend aus unterschiedlichen Polymeren, die sich in ihrem Schmelzpunkt unterscheiden, konnten Hybrid-Polymerrohre durch anschließendes Schmelzen des niedrigschmelzenden Polymerfadens hergestellt werden, bei denen die Ausrichtung der Polymerketten oder Molekülketten in den nicht geschmolzenen Polymerfäden erhalten bleibt. Zwar besitzen die Hybrid-Polymerrohre eine gesteigerte Zugfestigkeit, allerdings ist das Aufwickeln verschiedener Polymerfäden sehr aufwendig und erfordert einen mehrstufigen Prozess. Die Herstellung der Hybrid-Polymerrohre basiert in der Regel auf lösungsmittelbasierten Faserherstellungsprozessen. Für bestimmte medizinische Bereiche müssen die Restlösungsmittelgehalte allerdings so gering sein, das eine Herstellung auf Grundlage von lösungsmittelbasierten Verfahren nicht in Frage kommt. Ein weiterer Nachteil besteht darin, dass das Halbzeug für die Hybrid-Polymerrohre erst unmittelbar beim Aufwickeln vereint wird. Dadurch sind die Produktion der Rohre sowie die Lagerhaltung der unterschiedlichen Polymerfäden aufwendiger. Ein weiterer Nachteil des hier beschriebenen Verfahrens ist die starke Streuung der einzelnen Fasern der Hybridpolymerrohre, da das Aufwickelverhältnis der beiden Komponenten nicht exakt eingestellt werden kann.

Neben der Verwendung von reinen Polymeren für Stents offenbart WO 2007/021558 A2 den Einsatz von Faserverbundkunststoffe. Faserverbundkunststoffe bestehen aus Fasern, die entlang der Faserlängsachse eine hohe Festigkeit aufweisen, und einem Matrixpolymer, das in der Regel elastischer ist im Vergleich zur Polymerfaser. Für eine hohe radiale Festigkeit des Stents ist eine hohe Orientierung der Fasern notwendig. Die Stents aus Faserverbundkunststoffen werden durch gemeinsame Extrusion der Polymerfasern mit dem niedriger schmelzenden Matrixpolymer und anschließendem Streckblasformen (US 2008/0300670 A1) hergestellt. Durch das Streckblasformen wird zwar die Orientierung der Polymerfasern und damit die Festigkeit gesteigert, aber die Stents weisen nach der Implantation eine starke Kriechneigung auf, d.h. bei geringer Belastung ziehen sie sich wieder auf ihren ursprünglichen Durchmesser zusammen. Darüber hinaus ist die maximale Dehnung, die durch das Streckblasformen erreicht werden kann, durch die Differenz vom geringstmöglichen Innendurchmesser im Ausgangszustand und vom maximalen Außendurchmesser im aufgedehnten Zustand begrenzt.

Wie dargelegt weisen Polymere wie beispielsweise Polylactide als Stentmaterial den entscheidenden Nachteil einer begrenzten Festigkeit auf, weshalb Polymere in Form von Fasern zum Einsatz kommen. Faser enthaltende Stents, die eine geringe Orientierung der Fasern aufweisen, erreichen nur eine geringe Festigkeitssteigerung. Die Herstellung Faser enthaltender Stents, die eine hohe Orientierung der Fasern aufweisen, ist sehr aufwendig und nur über einen mehrstufigen lösungsmittelbasierten Prozess durchführbar. So hergestellte Stents besitzen einen hohen Restlösungsmittelgehalt und sind damit für den medizinischen Einsatz ungeeignet. Andere Methoden zur Festigkeitssteigerung wie z.B. das Verstrecken führen zu einer begrenzten Dehnbarkeit und damit zu einer starken Einschränkung bei der Verwendung der Biopolymerstents.

Ziel der vorliegenden Erfindung ist es daher, durch geeignete Maßnahmen und Verfahren die Festigkeit von Polymerrohren und daraus hergestellten Stents oder anderen Gefäßimplantaten zu steigern und Polymerrohre sowie Stents mit verbesserten Zugfestigkeiten und Rückstellkräften zum Nominaldurchmesser, d.h. dem Innendurchmesser des Stents im dilatierten, implantierten Zustand, bereitzustellen. Die erfindungsgemäße Herstellung von Polymerrohren beseitigt die starken Einschränkungen bzw. verbessert die Eigenschaften der Polymerrohre, die dadurch für die Herstellung von Stents besonders geeignet sind.

Aufgabe der vorliegenden Erfindung ist es ein Polymerrohr bereitzustellen, welches eine erhöhte radiale Festigkeit bei kaum verminderter Bruchdehnung aufweist und daher für die Herstellung von Stents besonders geeignet ist.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung, den Figuren sowie den Beispielen.

Die vorliegende Erfindung ist gerichtet auf ein **Polymerrohr** und vorzugsweise auf ein bioresorbierbares Polymerrohr, bestehend aus mindestens einem gerichtet aufgewickelten Mehrkomponentenfaden mit einem Durchmesser ≤ 200 µm, wobei der mindestens eine gerichtet aufgewickelte Mehrkomponentenfaden aus einer Faser-Komponente und einer Matrix-Komponente besteht.
Ein weiterer Aspekt der Erfindung ist ein Polymerrohr hergestellt durch gerichtetes Aufwickeln von mindestens einem Mehrkomponentenfaden, wobei der Mehrkomponentenfaden einen Durchmesser ≤ 200 µm und vorzugsweise einen Durchmesser im Bereich von 50 nm bis 200 µm aufweist.

Ein weiterer Aspekt der vorliegenden Erfindung betrifft ein Herstellungsverfahren umfassend den folgenden Schritt:
gerichtete Aufwicklung mindestens eines Mehrkomponentenfadens mit einem Durchmesser ≤ 200 µm, bestehend aus einer Faser-Komponente und einer Matrix-Komponente, zu einem Polymerrohr wie hierin offenbart. Dieses Polymerrohr besitzt noch Poren, welche erst im weiteren Verlauf entfernt werden.

Ein weiterer Aspekt der vorliegenden Erfindung betrifft Mehrkomponentenfäden mit einem Durchmesser ≤ 200 µm, zur Herstellung der hierin offenbarten Polymerrohre bestehend aus einer Faser-Komponente und einer Matrix-Komponente, wobei die Faser-Komponente entlang der Längsachse des Mehrkomponentenfadens verläuft und die Matrix-Komponente die Faser-Komponente umgibt und die Matrix-Komponente unter chemischen und/oder physikalischen Bedingungen plastisch verformbar ist unten denen die Faser-Komponente nicht plastisch verformt wird.

Bevorzugte Materialkombinationen für die Matrix-Komponente und die Faser-Komponente können ausgewählt werden aus der Gruppe bestehend aus:
Polyglycolid / PLLA, PDLLA / PLLA, PLLA / PLLA-Polyglycolid-Copolymer, PLLA-Polyglycolid-Copolymer / PLLA-Poly(ε-caprolacton)-Copolymer, PDLLA / PLLA-Polyglycolid-Copolymer, PLLA / Poly(ε-caprolacton) und Polyglycolid / Polyhydroxybutyrat.

Erfindungsgemäß enthalten die Mehrkomponentenfäden mit einem Durchmesser ≤ 200 µm mindestens eine Polymerfaser welche entlang der Längsachse des Mehrkomponentenfadens ausgerichtet ist.

Der hierin verwendete Begriff **"Polymerfaser"** bezieht sich auf Fasern, die aus langen Polymerketten oder Molekülketten bestehen. Fasern sind lange dünne Gebilde, die flexibel sind und entlang der Faserlängsachse hohe Zugkräfte aufnehmen können. Vorzugsweise sind die langen Polymerketten oder Molekülketten entlang der Faserlängsachse ausgerichtet. Die Polymerfasern bestehen somit aus langen Polymerketten oder Molekülketten, die in Faserrichtung orientiert sind. Die langen Polymerketten oder Molekülketten verlaufen möglichst gerade und parallel zueinander durch die Polymerfaser, d.h. sie weisen eine möglichst gestreckte Konformation auf. Sie sind nicht miteinander verknäult oder verwoben. Die Polymerfasern besitzen vorteilhafterweise eine hohe Anisotropie, die sich z.B. in einer hohen Festigkeit in Faserrichtung und einer geringen Festigkeit quer zur Faserrichtung ausdrückt. Die verwendeten Polymerfasern sind vorzugsweise Rundfasern, d.h. sie weisen einen kreisförmigen Querschnitt auf, können jedoch auch andere Querschnitte, wie oval oder eckig aufweisen.

Der hier verwendete Begriff **"Mehrkomponentenfaden"** bezieht sich auf fadenförmige Polymerverbunde, die mindestens eine Polymerfaser enthalten. Anders gesagt, bestehen die Mehrkomponentenfäden aus einer Faser-Komponente und einer Matrix-Komponente, wobei die Faser-Komponente und die Matrix-Komponente unterschiedliche chemische und/oder physikalische Eigenschaften aufweisen. Die Faser- und die Matrix-Komponente bestehen aus unterschiedlichen Polymeren. Die Faser-Komponente und die Matrix-Komponente unterscheiden sich dahingehend, dass der Mehrkomponentenfaden chemisch oder physikalisch so behandelt werden kann, beispielsweise durch Lösen oder Erhitzen, dass nur die Form und Orientierung der Faser-Komponente und der darin enthaltenen Polymerfaser oder Polymerfasern erhalten bleibt und die Matrix-Komponente ihre Form und ihre Eigenschaften verändert z.B. durch Auflösen oder Schmelzen. Somit kann z.B. die Matrix-Komponente aus einem oder mehreren Polymeren bestehen, die sich in einem Lösungsmittel gut lösen, in dem sich die Faser-Komponente nicht löst. Oder die Matrix-Komponente kann einen Schmelzpunkt besitzen, der unterhalb des Schmelzpunktes der Faser-Komponente liegt, sodass beim Erwärmen des Mehrkomponentenfadens nur die Matrix-Komponente schmilzt und nicht die Faser-Komponente.

So kann ein Mehrkomponentenfaden gemäß der vorliegenden Erfindung mit einem Lösungsmittel versetzt werden, worin sich die Matrix-Komponente teilweise oder vollständig löst und worin sich die Faser-Komponente nicht löst, wobei die Form und Orientierung der Faser-Komponente und der darin enthaltenen Polymerfaser oder Polymerfasern erhalten bleibt. Bei diesen Lösungsmitteln handelt es sich vorzugsweise um Trifluorethanol, Chloroform, Dichlormethan und Hexafluorisopropanol oder einem Gemisch aus zwei oder mehr der vorgenannten Lösungsmittel.

In einer bevorzugten Ausführungsform der vorliegenden Erfindung besteht der Mehrkomponentenfaden aus einer Faser-Komponente und einer Matrix-Komponente, wobei die Matrix-Komponente eine hohe Löslichkeit in mindestens einem organischen Lösungsmittel oder einem organischen Lösungsmittelgemisch aufweist.

Ebenfalls kann ein Mehrkomponentenfaden gemäß der vorliegenden Erfindung erhitzt werden, sodass nur die Matrix-Komponente schmilzt und nicht die Faser-Komponente, wobei die Form und Orientierung der Faser-Komponente und der darin enthaltenen Polymerfaser oder Polymerfasern erhalten bleibt.

In einer alternativen bevorzugten Ausführungsform besteht der Mehrkomponentenfaden aus einer Faser-Komponente und einer Matrix-Komponente, wobei die Matrix-Komponente einen Schmelzpunkt aufweist, der mindestens 10°C, weiter bevorzugt 20°C niedriger ist als der Schmelzpunkt der Faser-Komponente.

Ebenfalls bevorzugt ist eine Ausführungsform, in der der Mehrkomponentenfaden aus einer Faser-Komponente und einer Matrix-Komponente besteht, wobei die Matrix-Komponente eine hohe Löslichkeit in mindestens einem organischen Lösungsmittel oder einem organischen Lösungsmittelgemisch aufweist und wobei die Matrix-Komponente einen Schmelzpunkt aufweist, der mindestens 10°C, weiter bevorzugt 20°C niedriger ist als der Schmelzpunkt der Faser-Komponente.

Die Faser-Komponente kann aus einer Polymerfaser oder aus mehreren Polymerfasern bestehen. Die Polymerfasern der Faser-Komponente können aus unterschiedlichen Polymeren oder demselben Polymer bestehen. Die Polymerfasern der Faser-Komponente sind vorzugsweise entlang der Längsachse des Mehrkomponentenfadens ausgerichtet. Die Faser-Komponente muss nicht einen zusammenhängenden Bereich in dem Mehrkomponentenfaden einnehmen. Die Bereiche, die die Faser-Komponente einnimmt, können durch die Matrix-Komponente unterbrochen sein. Faser- und Matrix-Komponente sind vorzugsweise so im Mehrkomponentenfaden angeordnet, dass die Matrix-Komponente oder Teile davon durch die Faser-Komponente oder Teile davon nicht eingeschlossen sind. Die Faser- und Matrix-Komponenten sind beispielsweise so angeordnet, dass beim Versetzen des Mehrkomponentenfadens mit einem Lösungsmittel die Matrix-Komponente in Kontakt mit dem Lösungsmittel kommen kann.

Während die Faser-Komponente aus einer Polymerfaser oder mehreren Polymerfasern besteht, kann die Matrixkomponente eine beliebige Form innerhalb des Mehrkomponentenfadens aufweisen. Die Matrix-Komponente kann beispielsweise selbst eine Faserform besitzen oder aus mehreren Fasern bestehen, womit, anders formuliert, der Mehrkomponentenfaden aus zwei unterschiedlichen Fasern bestehen kann, nämlich der Faser-Komponente und der Matrix-Komponente in Faserform. Bevorzugt ist aber, wenn die Matrix-Komponente des Mehrkomponentenfadens keine Faserform aufweist, sondern, wenn die Faser-Komponente in die Matrix-Komponente des Mehrkomponentenfadens eingebettet ist.

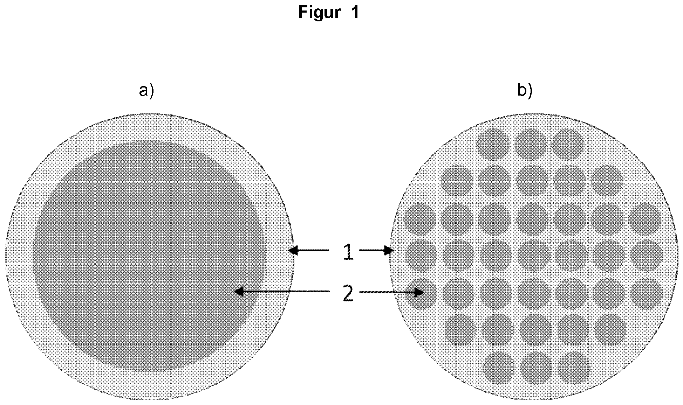

Der Querschnitt der Mehrkomponentenfäden kann ein beliebiges Profil haben, mit der Einschränkung, dass das Profil keinen Einschluss oder Einschlüsse der Matrix-Komponente über den gesamten Mehrkomponentenfaden aufweisen darf. Der Querschnitt kann entlang des Mehrkomponentenfadens konstant sein oder sich verändern, wenn beispielsweise eine Polymerfaser der Faser-Komponente endet und eine neue Polymerfaser anfängt. Das Profil des Querschnitts kann unter anderem ein Seite-an-Seite-, Segmented-Pie-, Kern-Mantel- oder Inseln-im-See-Profil sein. Bevorzugt ist jedoch, wenn der Querschnitt der Mehrkomponentenfäden ein Kern-Mantel-Profil oder ein Inseln-im-See-Profil aufweist. Der Querschnitt kann entlang des Mehrkomponentenfadens konstant sein oder sich verändern, wenn beispielsweise eine Polymerfaser der Faser-Komponente endet und eine neue Polymerfaser anfängt. Bei dem Kern-Mantel-Profil besteht der Mehrkomponentenfaden aus einer Polymerfaser der Faser-Komponente, die entlang des Mehrkomponentenfadens verläuft und die mit der Matrix-Komponente beschichtet oder ummantelt ist. Bei dem Inseln-im-See-Profil besteht die Faser-Komponente aus vielen einzelnen fibrillenartigen Polymerfasern ("Inseln"), die parallel entlang des Mehrkomponentenfadens verlaufen und die in der Matrix-Komponente eingebettet sind ("See", siehe Figuren 1a und 1b).

Der Durchmesser des erfindungsgemäß genutzten Mehrkomponentenfadens ist ≤ 200 µm und bevorzugt ≤ 150 µm, weiter bevorzugt ≤ 120 µm, weiter bevorzugt ≤ 100 µm, noch weiter bevorzugt ≤ 80 µm und besonders bevorzugt ≤ 50 µm. Der Durchmesser des Mehrkomponentenfadens liegt bevorzugt in folgenden Bereichen: zwischen 50 nm und 200 µm, weiter bevorzugt zwischen 100 nm und 150 µm, weiter bevorzugt zwischen 150 nm und 100 µm, noch weiter bevorzugt zwischen 200 nm und 80 µm und besonders bevorzugt zwischen 300 nm und 50 µm.

Das Verhältnis von Faser-Komponente zu Matrix-Komponente im Mehrkomponentenfaden kann beliebig gewählt werden. Ein hoher Faser-Komponenten-Anteil und damit auch ein hoher Anteil an ausgerichteten Polymerfasern führt zu einer großen Zugfestigkeit des Mehrkomponentenfadens und der daraus hergestellten Formen. Demnach ist es vorteilhaft, wenn der Mehrkomponentenfaden einen hohen Faser-Komponenten-Anteil aufweist. Bevorzugt ist daher, wenn der Mehrkomponentenfaden einen Faser-Komponenten-Anteil von ≥ 50% aufweist. Noch bevorzugter ist, wenn der Mehrkomponentenfaden einen Faser-Komponenten-Anteil von ≥ 60% aufweist und wesentlich bevorzugter ist, wenn der Mehrkomponentenfaden einen Faser-Komponenten-Anteil von ≥ 70% aufweist und am bevorzugtesten ist es, wenn der Mehrkomponentenfaden einen Faser-Komponenten-Anteil von ≥ 80% aufweist. Der Faser-Komponenten-Anteil liegt bevorzugt in folgenden Bereichen: zwischen 50% und 95%, weiter bevorzugt zwischen 60% und 90% und besonders bevorzugt zwischen 65% und 85%.

Der Begriff "resorbierbar" oder "bioresorbierbar" wie hierin verwendet bedeutet, dass der Mehrkomponentenfaden, respektive das Polymerrohr, oder der daraus hergestellte Stent, sich über eine gewisse Zeit in einem menschlichen oder tierischen Organismus langsam auflöst und irgendwann nur noch dessen Abbauprodukte im Körper vorliegen und vorzugsweise vom Körper ausgeschieden werden. Zu diesem Zeitpunkt sind feste Bestandteile oder Fragmente der Polymere nicht mehr vorhanden. Die Abbauprodukte sollten physiologisch weitgehend unbedenklich sein.

Erfindungsgemäß ist es bevorzugt, wenn die Faser-Komponente des Mehrkomponentenfadens hergestellt ist aus mindestens einem thermoplastischen Polymer und weiter bevorzugt aus einem thermoplastischen **bioresorbierbaren Polymer.** Thermoplastische bioresorbierbare Polymere können aus der folgenden Gruppe ausgewählt werden bestehend aus oder umfassend:
Poly(ε-caprolacton) (PCL), Polyurethan (PU), Polyhydroxybutyrate (PHB), Polylactonsäure, Polyglycolsäure (PGA), Polylactide, Polyglycolide, Copolymere der Polylactide und Polyglycolide, Polydioxanone, Polyethylenglycol (PEG), Polypropylencarbonat (PPC), Poly(desaminotyrosyl-tyrosine-ethylestercarbonat) (PDTE Carbonat).

Beispiele für nicht bioresorbierbare Polymere sind Nylon, Polymethylmethacrylat (PMMA), Polyamide (PA), Polycarbonat (PC), Polyethylenterephthalat (PET), Polypropylen (PP), Polystyren (PS), Polyethylen (PE), Silicone, Polytetrafluorethylen (PTFE), Polyvinylchlorid (PVC).
Da die Anwendung des Lösungsspinnens oder Elektrospinnens bevorzugt ist, sollten die bioresorbierbaren Polymere in einem organischen Lösungsmittel wie beispielsweise Aceton, Methanol, Ethanol, THF, Methylenchlorid, Benzol, Toluol, Chloroform, Trifluorethanol (TFE), Tetrachlorkohlenstoff, Ethylacetat, Diethylether, Acetonitril, 3-Pentanon, Butanon, Propanol, Hexafluoroisopropanol (HFIP) löslich sein. Ausgeschlossen von der vorliegenden Erfindung weil ungeeignet ist Collagen. Bevorzugt ist hingegen Polylactid wie z.B. PLLA sowie Copolymere aus Polylactid und Polyglycolid wie z.B. PLGA.

Erfindungsgemäß ist es bevorzugt, wenn die Matrix-Komponente des Mehrkomponentenfadens ebenfalls hergestellt ist aus mindestens einem thermoplastischen Polymer und weiter bevorzugt aus einem thermoplastischen **bioresorbierbaren Polymer.** Thermoplastische bioresorbierbare Polymere können aus der folgenden Gruppe ausgewählt werden bestehend aus oder umfassend:
Poly(ε-caprolacton), Polyurethan, Polyhydroxybutyrate, Polylactonsäure, Polyglycolsäure, Polylactide, Polyglycolide, Copolymere der Polylactide und Polyglycolide, Polydioxanone, Polyethylenglycol (PEG), Polypropylencarbonat (PPC), Poly(desaminotyrosyl-tyrosine-ethylestercarbonat) (PDTE Carbonat).

Dabei sollte das bioresorbierbare Polymer der Matrix-Komponente so gewählt werden, dass es sich mit der Faser-Komponente gut verbindet und entweder eine niedrigere Schmelztemperatur als die Faser-Komponente oder eine hohe Löslichkeit im Gegensatz zur Faser-Komponente aufweist. Vorzugsweise sind Faser-Komponente und Matrix-Komponente so zu wählen, dass sie ähnliche Degradationszeiten haben. Sie können aber auch unterschiedliche Degradationszeiten besitzen.

In einer bevorzugten Ausführungsform weist der Mehrkomponentenfaden eine der folgenden Faser-/Matrix-Komponenten-Zusammensetzungen auf:
Polyglycolid / PLLA, PDLLA / PLLA, PLLA / PLLA-Polyglycolid-Copolymer, PLLA-Polyglycolid-Copolymer / PLLA-Poly(ε-caprolacton)-Copolymer, PDLLA / PLLA-Polyglycolid-Copolymer, PLLA / Poly(ε-caprolacton) oder Polyglycolid / Polyhydroxybutyrat. Besonders bevorzugt ist, wenn die Faser-Komponente aus PLLA und die Matrix-Komponente aus PLLA-Polyglycolid-Copolymer bestehen.

Die Mehrkomponentenfäden können zu anderen Formen, wie z.B. Polymerrohre oder vor allem Stents verarbeitet werden, wobei die Form und Orientierung der Faser-Komponente und der darin enthaltenen Polymerfaser oder Polymerfasern erhalten bleibt.

Ein weiterer Aspekt der vorliegenden Erfindung betrifft die Verwendung von Mehrkomponentenfäden zur Herstellung von Polymerrohren. Weiterhin betrifft die vorliegende Erfindung die Verwendung Mehrkomponentenfäden zur Herstellung von Stents.

Die Verwendung von Mehrkomponentenfäden zur Herstellung von Polymerrohren hat sich gegenüber der Verwendung verschiedener Polymerfäden als vorteilhaft herausgestellt:
- So wird beim Mehrkomponentenfaden nur ein Polymerfaden hergestellt. Eine separate Polymerfäden-Herstellung, wie bei den Hybrid-Polymerrohren offenbart in WO 2015/091357 A1, entfällt damit. Zudem können die Polymerrohre schneller produziert werden, da keine separate Aufwicklung unterschiedlicher Polymerfäden notwendig ist.
- Der Mehrkomponentenfaden vereint die Faser-Komponente und die Matrix-Komponente bereits in einem gemeinsamen Halbzeug. Damit ist die Fertigung des Polymerrohrs aus einem Halbzeug möglich, wodurch nicht nur der Herstellungsprozess beschleunigt wird, sondern auch die Lagerhaltung vereinfacht wird.
- Beim Mehrkomponentenfaden können die beiden Polymere in einem exakten Mischungsverhältnis gleichzeitig verbunden werden. Im Gegensatz dazu tritt bei den Hybrid-Polymerrohren eine starke Streuung der einzelnen Fasern auf, da das Aufwickelverhältnis der beiden Polymerfäden nicht exakt eingestellt werden kann.
- Die Herstellung der erfindungsgemäßen Polymerrohre aus Mehrkomponentenfäden ist nicht auf lösungsmittelbasierte Prozesse beschränkt. Somit können Polymerrohre und Gefäßimplantate für medizinische Zwecke, wie z.B. Stents mit deutlich geringeren Restlösungsmittelgehalten erhalten werden.

Demnach ist die vorliegende Erfindung auch auf ein **Polymerrohr** und vorzugsweise auf ein bioresorbierbares Polymerrohr gerichtet, das aus mindestens einem aufgewickeltem Mehrkomponentenfaden besteht.

Ferner betrifft die vorliegende Erfindung **Polymerrohre** aus gerichtet aufgewickelten Mehrkomponentenfäden, wobei die Matrix-Komponente unter chemischen und/oder physikalischen Bedingungen plastisch verformbar ist unten denen die Faser-Komponente nicht plastisch verformt wird.

Das Polymerrohr aus dem mindestens einen gerichtet aufgewickelten Mehrkomponentenfaden besitzt im Gegensatz zum Polymerrohr nach der plastischen Behandlung Poren.

Als **Pore** wird ein Zwischenraum mit einem Volumen von mindestens **900 nm³** oder einem Maximaldurchmesser von mindestens **800 nm** bezeichnet. Als Maximaldurchmesser wird der größtmögliche Durchmesser in einer Pore beliebiger geometrischer Ausgestaltung bezeichnet. Poren beziehen sich dabei auf das innere Volumen des Rohres und nicht auf evtl. Unebenheiten an der Materialoberfläche, d.h. der inneren als auch äußeren Oberfläche des Polymerrohres. Der hier verwendete Begriff "Pore" bezieht sich dabei nicht auf Zwischenräume wie sie beispielsweise in Geweben oder Filzen oder Gespinsten von ungeordneten oder willkürlich ausgerichteten Fäden vorkommen. Die Fäden liegen dort ungeordnet aufeinander und die Zwischenräume zwischen den Polymerfäden. Selbst bei einem Verschmelzen dieser filzartigen oder gewebeartigen oder gespinstartigen Strukturen können die Poren nicht vollständig beseitigt werden, außer die Polymerfäden in diesen Strukturen werden vollständig verflüssigt, was jedoch auch die filzartigen oder gewebeartigen oder gespinstartigen Strukturen vollständig zerstört und daher widersinnig wäre.

Das erfindungsgemäße und vorzugsweise bioresorbierbare Polymerrohr besteht somit nicht aus einem Fadengewirr und besteht nicht aus einem Gewebe und besteht nicht aus einem Filz. Zudem bilden die aufgewickelten Mehrkomponentenfäden das Polymerrohr. Die Mehrkomponentenfäden werden nicht um ein Substrat oder ein Medizinprodukt wie z.B. einen Stent und vor allem einen Metallstent gewickelt, auf dem die aufgewickelten Mehrkomponentenfäden oder das erhaltene Polymerrohr verbleiben sollen. Die erfindungsgemäße Verwendung eines Dorns oder einer Spule zum Aufwickeln der Mehrkomponentenfäden dient nur dazu, dem Polymerrohr einen definierten Innendurchmesser, nämlich bereits den Nominaldurchmesser zu verleihen und ein gerichtetes Aufwickeln zu ermöglichen, wobei das fertige Polymerrohr natürlich von dem Dorn oder der Spule abgenommen wird. Die erfindungsgemäßen Polymerrohre umfassen daher kein Stützgerüst wie beispielsweise einen Stent, um den herum sich das Polymerrohr befindet. Das erfindungsgemäße Polymerrohr als auch die daraus vorzugsweise mittels Laser geschnittene Struktur bilden selbst den Stent.

In einer bevorzugten Ausführungsform ist das Polymerrohr hergestellt durch Aufwickeln mindestens eines Mehrkomponentenfadens, wobei der mindestens eine aufgewickelte Mehrkomponentenfaden einen Durchmesser ≤ 200 µm aufweist und aus einer Faser-Komponente und einer Matrix-Komponente besteht.

Anders formuliert, besteht das erfindungsgemäße Polymerrohr aus mindestens einem aufgewickelten Mehrkomponentenfaden, wobei die Faser-Komponente enthaltend in dem mindestens einen aufgewickelten Mehrkomponentenfaden aus mindestens einer Polymerfaser besteht, die entlang der Längsachse des mindestens einen aufgewickelten Mehrkomponentenfaden orientiert ist.

Der Begriff **"gerichtet"** wie hierin verwendet bezeichnet eine geordnete Aufwicklung des Mehrkomponentenfadens oder der Mehrkomponentenfäden. Eine gerichtete oder geordnete Aufwicklung des Mehrkomponentenfadens oder der Mehrkomponentenfäden kann die Aufwicklung nach einem bestimmten Muster bedeuten oder das Vorliegen einer bestimmten Ordnung zumindest in Teilbereichen oder Schichten des Polymerrohrs. Bevorzugt ist eine Aufwicklung wie ein Kabel auf einer Kabeltrommel. Eine gerichtete oder geordnete Aufwicklung schließt nicht aus, dass sich Mehrkomponentenfäden kreuzen oder übereinander liegen, wobei in gewissen Abschnitten oder Lagen oder Schichten des Polymerrohrs die aufgewickelten Mehrkomponentenfäden parallel zueinander liegen sollten. Nicht gerichtet oder nicht geordnet ist ein Aufbringen einer Lösung von Polymerfäden allgemein mittels Sprühvorrichtung oder Elektrospinning wie z.B. in US 2010/0070020 A1, WO 99/17817 A1, US 2004/0037813 A1 oder US 2013103139 A1 offenbart, weil sich dabei willkürliche Ausrichtungen der Polymerfäden ergeben. Da jedoch die Molekülketten oder synonym als Polymerketten bezeichnet, in der Polymerfaser bereits entlang der Längsachse des Mehrkomponentenfadens orientiert sind, kann diese Orientierung ausgenutzt werden, um vorteilhafte Eigenschaften zu erzeugen, wenn die Mehrkomponentenfäden ebenfalls in einer Ordnung oder Orientierung aufgewickelt oder zu einem Polymerrohr zusammengefügt werden. Das Gegenteil einer gerichteten oder geordneten Ausrichtung ist somit die willkürliche oder ungeordnete Ausrichtung.

Dem Begriff **"Aufwickeln"** oder "Aufwicklung" wie hierin verwendet kommt eine besondere Bedeutung zu. Um den mindestens einen Polymerfaden gerichtet aufwickeln zu können, wird der Mehrkomponentenfaden von der Spinndüse oder der Elektrospinndüse abgezogen. Die Spinndüse sprüht daher nicht den Mehrkomponentenfaden oder die Mehrkomponentenfäden auf den Dorn oder die Spule, wodurch ein Aufwickeln und erst recht ein gerichtetes Aufwickeln unmöglich ist, sondern produziert kontinuierlich einen Mehrkomponentenfaden, den man als einen Endlos- Mehrkomponentenfaden bezeichnen kann, der aufgewickelt wird. Der Mehrkomponentenfaden oder Endlos- Mehrkomponentenfaden verbindet sozusagen die Spinndüse mit dem Dorn oder der Spule. Auf dem Dorn oder der Spule wird daher ein Endlos- Mehrkomponentenfaden aufgewickelt. Es werden hingegen keine Vielzahl an Fadenstücken, verknäuelte Fäden, Fadengewirre oder ein Filz auf den Dorn aufgebracht oder gesprüht oder abgeladen, was nicht gerichtet erfolgen kann.

Der Begriff **"Wicklung",** wie hierein verwendet, bezeichnet dabei eine um eine Achse verlaufende Aufwicklung mindestens eines Mehrkomponentenfadens. Mehrlagige Wicklungen bestehen aus konzentrischen Wendeln, die übereinander liegen. Alle Wicklungen des mindestens einen Mehrkomponentenfadens, die in einer Ebene parallel zur Achse des Polymerrohrs verlaufen, bilden eine Schicht in der Wand des Polymerrohrs.

Anders ausgedrückt, handelt es sich bei dem Polymerrohr gemäß der vorliegenden Erfindung um eine Wicklung mindestens eines Mehrkomponentenfadens.

Durch das gerichtete Aufwickeln des Mehrkomponentenfadens verläuft dieser vorzugsweise in Umfangsrichtung und besitzt vorzugsweise ein geringes radiales Profil. Unter dem Begriff **"radiales Profil"** kann der unterschiedliche Abstand des Mehrkomponentenfadens zu einer Achse, um die die Aufwicklung erfolgt, verstanden werden. Die gerichtete Aufwicklung sollte entlang des Umfangs so erfolgen, dass das radiale Profil des Mehrkomponentenfadens minimal ist. Demnach ist es bevorzugt, dass die gerichtete Aufwicklung parallel zur Achse des Polymerrohrs durchgeführt wird, d.h. der mindestens eine Mehrkomponentenfaden in Lagen aufgewickelt wird. Erfolgt die Aufwicklung größtenteils lagenweise, dann weist der aufgewickelte mindestens eine Mehrkomponentenfaden ein geringes radiales Profil auf.

Die Polymerrohre vor der plastischen Behandlung besitzen noch Poren zwischen dem gerichtete aufgewickelten Mehrkomponentenfaden oder den gerichtete aufgewickelten Mehrkomponentenfäden, welche durch plastische Behandlung entfernt werden. Dann ergeben sich porenfreie Polymerrohre bestehend aus einem Verbund aus mindestens einer Polymerfaser und mindestens einem Matrixpolymer, wobei die mindestens eine Polymerfaser in dem mindestens einen Matrixpolymer eingebettet ist, in Umfangsrichtung verläuft, und die mindestens eine Polymerfaser ein geringes radiales Profil aufweist.

Der Begriff **"porenfrei"** wird wie folgt definiert: Ein herkömmlich extrudiertes Rohr gleichen Materials wird im Außendurchmesser und in der Wandstärke vermessen. Über den gemittelten Außendurchmesser und die gemittelte Wandstärke, sowie die gemessene Länge des Rohrs wird das Volumen ermittelt. Weiterhin wird die Masse des Rohrs auf einer Feinwaage bestimmt. Die Masse des Rohrs wird durch das Volumen geteilt. Dadurch ergibt sich die mittlere Dichte des Rohrs. Das erfindungsgemäß hergestellte Rohr wird in gleicher Weise vermessen und ebenfalls die mittlere Dichte bestimmt. Sofern die mittlere Dichte des extrudierten Polymerrohrs maximal 5%, bevorzugt nicht größer als 3% größer als die mittlere Dichte des erfindungsgemäß hergestellten Rohrs ist, so wird das erfindungsgemäße Rohr als "porenfrei" bezeichnet. Ein Polymerrohr ist daher "porenfrei", wenn seine mittlere Dichte um weniger als 5% kleiner, bevorzugt um weniger als 3% kleiner der mittleren Dichte eines mittels Extrusion hergestellten Polymerrohres gleichen Materials ist.

Somit betrifft die vorliegende Erfindung ein porenfreies Polymerrohr und vorzugsweise ein bioresorbierbares porenfreies Polymerrohr hergestellt durch Aufwicklung mindestens eines Mehrkomponentenfadens und der aufgewickelte mindestens eine Mehrkomponentenfaden vereinigt ist oder genauer gesagt porenfrei vereinigt ist.

Somit betrifft die vorliegende Erfindung ein porenfreies Polymerrohr und vorzugsweise ein bioresorbierbares porenfreies Polymerrohr hergestellt aus einem Polymerrohr durch porenfreie Vereinigung des aufgewickelten mindestens einen Mehrkomponentenfadens.

Ferner betrifft die vorliegende Erfindung ein porenfreies und vorzugsweise bioresorbierbares Polymerrohr hergestellt durch gerichtete Aufwicklung mindestens eines Mehrkomponentenfadens, wobei der mindestens eine Mehrkomponentenfaden mit einem Durchmesser von ≤ 200 µm aus einer Faser-Komponente und einer Matrix-Komponente besteht und der gerichtet aufgewickelte mindestens eine Mehrkomponentenfaden vereinigt ist oder genauer gesagt porenfrei vereinigt ist.

Zudem betrifft die vorliegende Erfindung ein bioresorbierbares porenfreies Polymerrohr hergestellt durch Aufwicklung mindestens eines Mehrkomponentenfadens, wobei der mindestens eine Mehrkomponentenfaden mit einem Durchmesser von ≤ 200 µm aus einer Faser-Komponente und einer Matrix-Komponente besteht und der aufgewickelte mindestens eine Mehrkomponentenfaden unter Beibehaltung der Ausrichtung der Polymerfaser oder Polymerfasern der Faser-Komponente entlang der Fadenlängsachse vereinigt ist oder genauer gesagt porenfrei vereinigt ist.

Alternativ betrifft die vorliegende Erfindung ein porenfreies Polymerrohr und bevorzugt ein bioresorbierbares porenfreies Polymerrohr hergestellt durch gerichtete Aufwicklung mindestens eines Mehrkomponentenfadens, wobei der mindestens eine Mehrkomponentenfaden mit einem Durchmesser von ≤ 200 µm aus einer Faser-Komponente und einer Matrix-Komponente besteht und der gerichtet aufgewickelte mindestens eine Mehrkomponentenfaden unter Beibehaltung der Ausrichtung der Polymerfasern entlang der Fadenlängsachse vereinigt ist oder genauer gesagt porenfrei vereinigt ist.

Ein weiterer Aspekt der vorliegenden Erfindung betrifft porenfreie Polymerrohre bestehend aus einer Faser-Komponente und einer Polymermatrix, wobei die Faser-Komponente eingebettet ist in der Polymermatrix, ein geringes radiales Profil aufweist und in Umfangsrichtung verläuft.

Erfindungsgemäß werden die aufgewickelten oder die gerichtet aufgewickelten Mehrkomponentenfäden vereinigt, so dass die Poren entfernt werden und ein porenfreies massives Polymerrohr resultiert, ohne dass die Orientierung der in der Faser-Komponente enthaltenen Polymerfaser oder Polymerfasern verändert wird. Die Vereinigung wirkt bevorzugt nicht auf die Faser-Komponente des mindestens einen aufgewickelten Mehrkomponentenfadens, sondern nur auf die Matrix-Komponente. Bei der Vereinigung bleibt die Ausrichtung der Polymerketten oder Molekülketten entlang der Längsachse des Mehrkomponentenfadens vorzugsweise erhalten. Eine derartige Ausrichtung oder Orientierung der Molekülketten oder der Polymerketten kann jedoch durch ein Extrusionsverfahren oder Spritzgussverfahren nicht erhalten werden. Die unterschiedlichen chemischen und/oder physikalischen Eigenschaften der Faser-Komponente und der Matrixkomponente des mindestens einen aufgewickelten Mehrkomponentenfadens werden bei der Vereinigung genutzt, um ein porenfreies massives Polymerrohr unter Erhalt der Ausrichtung der Polymerfaser oder Polymerfasern enthaltend in der Faser-Komponente, zu erhalten.

Die erfindungsgemäßen porenfreien Polymerrohre weisen bevorzugt einen Innendurchmesser von 0,25 - 20 mm, weiter bevorzugt 0,5 - 15 mm, noch weiter bevorzugt von 1 - 10 mm und besonders bevorzugt von 1,5 - 5 mm auf. Beispielsweise werden für Stents der koronaren Gefäße typischerweise Rohre mit einem Innendurchmesser von 2,0 bis 4,0 mm verwandt mit Wandstärke von 100 bis 200 µm. Entsprechend betragen die Außendurchmesser der porenfreien Polymerrohre 2,2 bis 4,4 mm.

Das Rohr ist vorzugsweise aus resorbierbaren Polymeren hergestellt. Die Polymerrohre besitzen bevorzugt einen kreisrunden Querschnitt. Der Begriff "Wand" oder "Rohrwand", wie hierin verwendet, bezeichnet die Mantel- bzw. Zylinderfläche des Polymerrohrs und somit den durch Aufwickeln mindestens eines Polymerfadens erhaltenen Anteil des Polymerrohrs ohne eventuell zusätzlich aufgebrachte Beschichtungen. Ein erfindungsgemäßes Polymerrohr weist nur eine nahtlose Wand auf. Der Begriff "Wanddicke" oder auch "Wandstärke", wie hierin verwendet, bezeichnet die Differenz der inneren und der äußeren Abmessungen, also des Außen- und des Innendurchmessers des Rohres.

Ein weiterer Aspekt der Erfindung bezieht sich auf Polymerrohre hergestellt aus mindestens einem Mehrkomponentenfaden, wobei der Mehrkomponentenfaden einen Durchmesser vorzugsweise im Bereich von 50 nm bis 200 µm aufweist und die Mehrkomponentenfäden aus unterschiedlichen, vorzugsweise bioresorbierbaren Polymeren bestehen. Ein weiterer Aspekt der Erfindung bezieht sich auf Polymerrohre hergestellt aus mehreren Mehrkomponentenfäden, wobei die Polymerfäden einen Durchmesser vorzugsweise im Bereich von 50 nm bis 200 µm aufweisen und die Mehrkomponentenfäden aus unterschiedlichen, vorzugsweise bioresorbierbaren Polymeren bestehen. Die erfindungsgemäße Herstellung der Polymerrohre durch Aufwicklung von Mehrkomponentenfäden lässt die Kombination von unterschiedlichen Faser-Komponenten und Matrix-Komponenten in den Polymerrohren zu, wobei die unterschiedlichen Faser-Komponenten und Matrix-Komponenten in einer gezielten und für das Endprodukt vorteilhaften Anordnung vorliegen können. Beispielsweise können Polymerrohre aus verschiedenen Mehrkomponentenfäden mit unterschiedlichen Faser-Komponenten und gleichen Matrix-Komponenten aufgewickelt werden, sodass Polymerrohre entstehen, die aus verschiedenen Polymerfasern bestehen und eine vorteilhafte Zugfestigkeit aufweisen, die mit einem aufgewickelten Mehrkomponentenfaden nicht erzielt werden kann. Dadurch können dem erfindungsgemäßen Polymerrohr gezielt Eigenschaften verschiedener Faser-Komponenten und Matrix-Komponenten verliehen werden. Mit der Wahl der unterschiedlichen Mehrkomponentenfäden sind daher Polymerrohre herstellbar, die genau auf die Verwendung abgestimmt werden. Erfindungsgemäß bevorzugt ist die Verwendung zur Herstellung von Stents oder anderer Gefäßprothesen. In diesem Fall können die Polymerrohre genau auf die physiologischen Eigenschaften der humanen Gefäße bzw. die Heilungsprozesse und Degradationsvorgänge abgestimmt werden.

So können die Rohre z.B. lagenweise aus unterschiedlichen Mehrkomponentenfäden bestehen. Die innere (dem Lumen zugewandte) und äußere (der Gefäßwand zugewandte) Schicht der Wand eines erfindungsgemäßen Polymerrohres kann bevorzugt aus einem Polymer mit sehr langsamer Degradation bestehen, während dazwischen im Inneren der Wand (im Inneren des Rohrquerschnittes) ein Polymer mit hoher Festigkeit (aber gegebenenfalls schneller Degradation) verwendet wird.

Neben der lagenweisen Kombination unterschiedlicher Mehrkomponentenfäden in der Wand der Polymerrohre (radiale Polymerkombinationen) sind erfindungsgemäß auch Polymerrohre herstellbar, die über die Rohrlänge unterschiedliche Mehrkomponentenfäden und damit unterschiedliche Eigenschaften aufweisen (axiale Polymerkombinationen). Das heißt die unterschiedlichen Mehrkomponentenfäden können sowohl in Schichten als auch in Ringen in der Wand des Polymerrohrs variieren oder angeordnet sein.

Jegliche Kombinationen von unterschiedlichen Mehrkomponentenfäden in radialer und axialer Richtung sind möglich. Dabei entspricht es einer Ausführungsform, dass die Polymerrohre aus einem Mehrkomponentenfaden hergestellt werden und der Mehrkomponentenfaden aus einer Faser-Komponente und einer Matrix-Komponente besteht. Die Erfindung umfasst aber auch Polymerrohre, hergestellt aus mindestens zwei Mehrkomponentenfäden bestehend aus unterschiedlichen Faser- und/oder Matrix-Komponenten, bevorzugt bestehend aus resorbierbaren Polymeren.

Es ist des Weiteren bevorzugt, wenn die erfindungsgemäßen Polymerrohre eine Wandstärke ≤ 250 µm (z.B. für periphere Gefäße), weiter bevorzugt ≤ 200 µm, weiter bevorzugt ≤ 150 µm (z.B. für koronare Gefäße), und besonders bevorzugt ≤ 100 µm aufweisen. Ein weiterer Aspekt der Erfindung betrifft Polymerrohre, die über die Rohrlänge zu unterschiedlichen Wandstärken aufgewickelt werden. Diese Wandstärken sollen den Festigkeitsbeanspruchungen der, aus den erfindungsgemäßen Polymerrohren hergestellten, Bauteilen entsprechen.

Die erfindungsgemäßen Polymerrohre weisen mindestens 1, bevorzugt mindestens 2 und noch weiter bevorzugt mindestens 3 Schichten bzw. Lagen von Mehrkomponentenfäden auf. Dabei besteht eine Schicht aus den Wicklungen des mindestens einen Mehrkomponentenfadens, die in einer Ebene verlaufen, die parallel zur Achse des Polymerrohrs liegt, d. h. die Dicke einer Schicht ist dabei in der Regel etwas geringer als der Durchmesser des verwendeten Mehrkomponentenfadens.

Bei den erfindungsgemäß hergestellten Polymerrohren weisen die Polymerfasern der Faser-Komponente in den Mehrkomponentenfäden vorzugsweise besonders lange Molekülketten auf, welche zudem entlang der Faserlängsachse ausgerichtet sind, insbesondere wenn die Mehrkomponentenfäden durch Lösungsspinnen hergestellt werden. Dies ist für die Festigkeit und eine langsamere Degradation der Polymerrohre von Vorteil. Durch die Ausrichtung der Molekülketten entlang der Faserlängsachse baut sich bei Deformation des Polymerrohres oder eines aus dem Polymerrohr hergestellten Stents eine Rückstellkraft auf, welche darauf gerichtet ist, das Rohr bzw. den Stent wieder auf den Außendurchmesser des Dorns für die Aufwicklung zu bringen, d.h. auf den Nominaldurchmesser. Dabei entspricht der Außendurchmesser des Dorns dem Innendurchmesser des Polymerrohrs oder des Stents nach dem Vereinigen der ausgerichteten Molekülketten. Es ist erfindungsgemäß, dass das Polymerrohr und damit auch das porenfreie Polymerrohr sowie der aus dem porenfreien Polymerrohr vorzugsweise durch Laserschneiden hergestellte Stent im Nominaldurchmesser hergestellt werden, d.h. der Außendurchmesser des Dorns entspricht dem Innendurchmesser des Polymerrohrs nach der Dilatation, damit beim gekrimpten Polymerrohr oder beim gekrimpten Stent eine Rückstellkraft in Richtung des Nominaldurchmessers aufgebaut wird. Im Stand der Technik werden derartige Polymerrohre häufig durch Extrudieren hergestellt, wobei bei der Extrusion keine Ausrichtung der Molekülketten in radialer Richtung erfolgt, wodurch extrudierte Rohre im Vergleich zu den erfindungsgemäßen Rohren deutlich geringere radiale Zugfestigkeiten und Bruchdehnungen aufweisen und sich aufgrund der ungeordneten und willkürlichen Anordnung der Molekülketten keine oder nur eine deutlich geringere Rückstellkraft in Richtung Nominaldurchmesser aufbaut.

Eine bevorzugte Ausführungsform der Erfindung betrifft ein porenfreies Polymerrohr, welches mindestens einen **Wirkstoff** umfasst. Bevorzugt ist dabei, dass sich der mindestens eine Wirkstoff in der Wand des Polymerrohrs befindet. Es handelt sich dabei bevorzugt um Wirkstoffe, die zur Verminderung einer Restenose geeignet sind. Geeignete Wirkstoffe sind insbesondere antiproliferative oder antirestenotische Wirkstoffe.

Der mindestens eine eingesetzte antiproliferative oder antirestenotische Wirkstoff ist bevorzugt ausgewählt aus der Gruppe umfassend oder bestehend aus: Paclitaxel, Rapamycin und deren Derivaten, wie 6-α-Hydroxy-Paclitaxel, Baccatin oder andere Taxotere, Biolimus A9, Myolimus, Novolimus, Pimecrolimus, Tacroliums, Temsirolimus, Zotarolimus, Everolimus, Ridaforolimus oder weiteren sogenannten "Limus"-Derivate (auch Rapalogs genannt).

Es können auch Wirkstoffkombinationen verwendet werden. Dabei kann die Konzentration der Wirkstoffe an verschiedenen Stellen der erfindungsgemäß hergestellten Polymerrohre variieren, so kann an den Enden des Rohrs mehr Wirkstoff enthalten sein als im mittleren Bereich. Die Wirkstoffkonzentration kann auch im Verlauf der Wandstärke variieren und zum Beispiel einen Gradienten aufweisen, so dass auf der äußeren Oberfläche (abluminale Oberfläche) des Polymerrohrs mehr Wirkstoff vorliegt als auf der Oberfläche im Inneren des Rohrs (luminale Oberfläche) oder umgekehrt.

Zur besseren **Röntgensichtbarkeit** der implantierten Stents im Körper werden entweder Werkstoffe mit höherer Dichte, d.h. höherer Röntgensichtbarkeit für die Herstellung der Stents verwandt (z.B. beim Boston Scientific "Element"-Stent der Werkstoff PtCr) oder bevorzugt sogenannte Röntgenmarker in die proximalen und distalen Enden der Stents eingesetzt. Die Röntgenmarker bestehen z.B. aus Tantal, Platin oder seltener Gold, werden in kleine Markerösen eingecrimpt und häufig auch zusätzlich laserverschweißt.

Bei den erfindungsgemäßen Polymerrohren bestehen mehrere Möglichkeiten, die Röntgensichtbarkeit herzustellen. Die **Röntgenmarker** können als kleine runde Pellets oder Scheiben (ähnlich den derzeitigen Röntgenmarkern) oder auch als dünne Folien oder Drähte beim Wickeln der Rohre mit in die Rohrwand eingearbeitet, d.h. eingebunden werden.

Die bevorzugte Methode ist aber, bereits bei der Herstellung des dünnen Mehrkomponentenfadens **Röntgenmarker** wie z.B. Pulver aus röntgensichtbaren Werkstoffen wie Wolfram, Tantal, Platin, Gold in die Matrix-Komponente und/oder Faser-Komponente des Mehrkomponentenfadens einzumischen. Beim Aufwickeln werden die röntgensichtbaren Werkstoffe somit über die gesamte Rohrwandstärke oder bevorzugt nur in bestimmte Schichten und Lagen und/oder bestimmten Bereichen des Polymerrohres eingebaut. Die Pulver haben eine Partikelgröße von kleiner 10 µm, vorzugsweise kleiner 5 µm und noch weiter bevorzugt kleiner 1 µm. Die Pulver bzw. die Röntgenmarker werden bei der Degradation des implantierten Polymerstents herausgelöst. Aufgrund ihrer Partikelgröße und ihrer Bioverträglichkeit (mikrobiellen Inertheit) sind die Röntgenmarker für den menschlichen Organismus unbedenklich.

Bei Einbringung der röntgensichtbaren Pulver oder der Röntgenmarker z.B. nur in den inneren Kern der Polymerrohrwand wird sichergestellt, dass bis zur nahezu vollständigen Degradation des Stents die Röntgensichtbarkeit erhalten bleibt. Werden die röntgensichtbaren Pulver dagegen nur in die äußeren Randschichten der Rohre (nicht in den Kern der Rohrwand) eingebracht, kann der Degradationsfortschritt, d.h. der Abbau der Stents z.B. anhand der verbliebenen Röntgensichtbarkeit ermittelt werden.

Eine weitere Ausführungsform ist, dass die röntgensichtbaren Pulver oder die Röntgenmarker nicht über die gesamte Rohrlänge, sondern nur in gewissen Abständen ringförmig eingebracht werden. Die Abstände der röntgensichtbaren Ringe sollten vornehmlich der Stentlänge entsprechen, die aus den Rohren lasergeschnitten wird. Auf diese Weise erhält man Stents mit einer Röntgensichtbarkeit nur an den proximalen und distalen Stentenden.

Jede andere mögliche Ausführungsform, d.h. Einbringung der röntgensichtbaren Pulver oder der Röntgenmarker in das Polymerrohr ist denkbar und möglich.

Anstelle oder zusätzlich zu den aufgeführten Wirkstoffen oder Röntgenmarkern können auch **Partikel oder Nanopartikel** mit physikalischen (z.B. magnetischen oder radioaktiven) und/oder chemischen (z.B. zur pH-Wert Änderung) und/oder physiologischen (z.B. antibakteriellen) Wirkungsweisen in die Rohrwandung eingebracht werden.

Eingebrachte radioaktive Partikel können z.B. der Vermeidung einer zu starken Proliferation bei der Gefäßheilung dienen. Chemische Partikel können z.B. bei Stents aus Polylactiden zu einer Pufferung des pH-Wertes beitragen, da das resorbierbare Material zu sauren Milchsäureabbauprodukten zerfällt.

Ebenso können zusätzlich kurze Fäden eines anderen polymeren oder metallischen Werkstoffes zur Festigkeitssteigerung in das Polymerrohr eingebracht werden. Diese festigkeitssteigernden Fäden können ebenfalls bioresorbierbar oder aber auch biostabil sein. Insbesondere wenn die zusätzlichen Fäden aus einem nicht biodegradierbaren Werkstoff bestehen, sollten die Fadenlängen vorzugsweise weniger als 50 µm, weiter bevorzugt weniger als 20 µm und besonders bevorzugt 10 Mikrometer und weniger betragen.

Grundsätzlich lassen sich durch die erfindungsgemäße Herstellung der Polymerrohre aus Mehrkomponentenfäden und der Zugabe von Additiven Rohre mit vielfältigen Wirkmechanismen und Eigenschaften herstellen.

Zur Herstellung von glatten Rohrstrukturen innen und/oder außen kann eine zusätzliche polymere Trennschicht verwendet werden. Für die Innenseite wird der Mehrkomponentenfaden nach der Aufbringung der Trennschicht auf diese aufgewickelt. Zur Aufbringung auf der Außenseite wird die Trennschicht nach dem Aufwickeln des Mehrkomponentenfadens aufgebracht. Diese polymere Trennschicht kann durch Aufwickeln von Fasern, Aufsprühen, Tauchbeschichten oder durch jegliches anderes Verfahren sowohl für innen als auch außen aufgebracht werden.

Die vorliegende Erfindung betrifft zudem **Verfahren zur Herstellung eines Polymerrohrs,** bevorzugt eines (bio)resorbierbaren Polymerrohrs, umfassend mindestens den folgenden Schritt:
Aufwicklung mindestens eines Mehrkomponentenfadens mit einem Durchmesser ≤ 200 µm, bestehend aus einer Faser-Komponente und einer Matrix-Komponente zu einem Polymerrohr oder einem noch nicht porenfreien Polymerrohr.

Erfindungsgemäß kann dieses Verfahren folgenden weiteren Schritt umfassen: plastische Verformung der Matrix-Komponente unter chemischen und/oder physikalischen Bedingungen unten denen die Faser-Komponente nicht plastisch verformbar ist zwecks Herstellung eines porenfreien Polymerrohrs und laserschneiden eines Stents aus dem porenfreien Polymerrohr.

Die plastische Verformung der Matrix-Komponente erfolgt vorzugsweise lösungsmittelbasiert durch Einwirkung von Trifluorethanol oder Chloroform oder

Dichlormethan oder Hexafluorisopropanol oder eines Gemisches aus zwei oder mehr der vorgenannten Lösungsmittel.

Der Durchmesser des Mehrkomponentenfadens ist dabei ≤ 200 µm und bevorzugt ≤ 150 µm, weiter bevorzugt ≤ 120 µm, weiter bevorzugt ≤ 100 µm, noch weiter bevorzugt ≤ 80 µm und besonders bevorzugt ≤ 50 µm. Die erfindungsgemäß verwendeten Mehrkomponentenfäden sind bevorzugt Rundfasern, das heißt sie weisen einen runden Querschnitt auf, können jedoch auch andere Querschnitte, wie oval oder eckig aufweisen.

Erfindungsgemäß ist es bevorzugt, wenn der mindestens eine Mehrkomponentenfaden in Schritt a) auf einem sich drehenden Dorn aufgewickelt wird. Der Dorn weist bevorzugt einen konstanten Durchmesser auf, kann aber auch konisch oder wellenförmig ausgeformt sein, so dass der Durchmesser des Polymerrohrs bei gleicher Wandstärke über dessen Länge variiert. Alternativ kann über die Länge des Rohrs auch dessen Wandstärke variieren, so dass bei gleichbleibendem Innendurchmesser der Außendurchmesser variiert. Bei erfindungsgemäßen Polymerrohren, die für die Herstellung von Stents verwendet werden, ist es besonders bevorzugt, wenn zumindest der Innendurchmesser über die gesamte Länge des Polymerrohrs konstant ist.

Der mindestens eine Mehrkomponentenfaden wird erfindungsgemäß gerichtet aufgewickelt. Dadurch erhalten in bestimmten Bereichen des Polymerrohrs oder in bestimmten Lagen oder über das gesamte Polymerrohr die Mehrkomponentenfäden eine vorgegebene Ausrichtung, wobei dadurch wiederum die Molekülketten verschiedener Polymerfasern der Faser-Komponente, welche entlang der Längsachse des jeweiligen Mehrkomponentenfäden ausgerichtet sind, zueinander auch eine bestimmte Ausrichtung erhalten, wie z.B. konische Lagen oder zylindrische Lagen um die Längsachse des Polymerrohrs. Durch dieses gerichtete Aufwickeln der Mehrkomponentenfäden werden dem Polymerrohr vorteilhafte Eigenschaften verliehen, wie beispielsweise eine höhere Zugfestigkeit sowie eine höhere Formstabilität im Nominaldurchmesser, als auch eine Rückstellkraft eines deformierten Polymerrohres oder eines daraus hergestellten deformierten Stents, wobei diese Rückstellkraft das deformierte Polymerrohr als auch den deformierten Stent wieder auf den Nominaldurchmesser bringt, sei es aus einer überdehnten oder einer zusammengedrückten Form. Daher ist auch wichtig, dass das Polymerrohr bei der Herstellung bereits den Nominaldurchmesser besitzt, d.h. die Mehrkomponentenfäden auf einem Dorn oder einer Spule aufgewickelt werden, welcher oder welche einen Außendurchmesser hat, der dem Innendurchmesser des Polymerrohrs oder des daraus hergestellten Stents entspricht, den das Polymerrohr oder der Stent nach der Dilatation im Gefäß haben soll.

Der Mehrkomponentenfaden wird bevorzugt unter konstanter Kraft abgelegt. Die Kraft ist dabei bevorzugt > 1 N, weiter bevorzugt > 3 N, noch weiter bevorzugt > 5 N. Die Kraft darf die maximale Zugfestigkeit multipliziert mit der Mehrkomponentenfadenquerschnittsfläche nicht überschreiten, sonst reißt der Faden.

Es ergibt sich für die Herstellung eines Polymerrohrs und vorzugsweise eines bioresorbierbaren porenfreien Polymerrohrs ein bevorzugtes Verfahren, welches den folgenden Schritt umfasst:
Aufwickeln oder gerichtetes Aufwickeln mindestens eines Mehrkomponentenfadens mit einem Durchmesser von ≤ 200 µm auf einen sich drehenden Dorn zu einem Polymerrohr.

Es ist zudem bevorzugt, wenn der Mehrkomponentenfaden, welcher erfindungsgemäß zu Polymerrohren aufgewickelt wird, durch Lösungsspinnen oder Schmelzspinnen hergestellt wird. Beim Lösungsspinnen wird das Polymer durch ein Lösungsmittel verflüssigt, beim Schmelzspinnen durch Erwärmung. Besonders bevorzugt sind erfindungsgemäße Verfahren, wobei der mindestens eine Mehrkomponentenfaden durch Lösungsspinnen oder Schmelzspinnen hergestellt und gleichzeitig auf einem sich drehenden Dorn aufgewickelt wird. Es ist auch bevorzugt, wenn es sich bei dem verwendeten Mehrkomponentenfaden um einen unendlich fortlaufenden Faden oder Strang handelt. Dieser unendlich fortlaufende Mehrkomponentenfaden wird nach Erreichen der gewünschten Wandstärke auf dem Dorn abgeschnitten.

Eine besonders bevorzugte Form des Lösungsspinnens ist das Elektrospinnen. Beim Elektrospinnen werden sehr dünne Fasern oder Fäden aus Polymerlösungen durch die Behandlung in einem elektrischen Feld hergestellt. Hierbei wird die Polymerlösung an einer Elektrode dosiert und durch das elektrische Feld von der Elektrode abgezogen und beschleunigt. Das Lösungsmittel verdampft bei dem extrem dünnen Faden sehr schnell und es kommt zu einer Ordnung der Polymerketten zu Kristalliten (Polymerkristallisation aus der Lösung). Bevorzugte Mehrkomponentenfäden, die mit dem Elektrospinnen hergestellt werden können, sind Kern-Mantel-Fäden. Das mindestens eine Faserpolymer der Faser-Komponente wird durch eine innere Düse und das mindestens eine Matrixpolymer der Matrix-Komponente durch einen äußeren, die innere Düse umschließenden Ring ausgepresst.

Auf dem sich drehenden Dorn wird dabei der vorzugsweise unendlich fortlaufende Mehrkomponentenfaden, bevorzugt aus resorbierbaren Polymeren, durch eine Apparatur gleichmäßig bis zu einer vorbestimmten Wandstärke aufgewickelt. Man erhält ein aus einem Mehrkomponentenfaden gewickeltes Rohr, wobei der Faden bzw. die Fäden idealer Weise perfekt parallel zueinander liegen. Dabei ist es besonders bevorzugt, dass der mindestens eine Mehrkomponentenfaden so aufgewickelt wird, dass eine Mantelfläche bzw. Wand des Polymerrohrs entsteht, die lückenlos bzw. durchgehend ist. Das erfindungsgemäße Polymerrohr weist daher bevorzugt keine Unterbrechung auf.

Offenbart werden zudem **Verfahren zur Herstellung eines** porenfreien **Polymerrohrs,** bevorzugt eines (bio)resorbierbaren Polymerrohrs, umfassend mindestens den folgenden Schritt:
porenfreies Vereinigen des mindestens einen aufgewickelten Mehrkomponentenfadens.

Die Poren zwischen den Fäden werden durch anschließendes Vereinigen der Fäden geschlossen. Das Vereinigen der Fäden erfolgt so, dass die Ausrichtung der Polymer- und Molekülketten der Polymerfasern enthaltend in der Faser-Komponente nicht verloren geht und ein massives porenfreies Polymerrohr entsteht. Bevorzugt ist, wenn das Vereinigen des mindestens einen aufgewickelten Mehrkomponentenfadens lösungsmittelbasiert oder durch Schmelzen erfolgt.

Bei dem lösungsmittelbasierten Vereinigen werden die unterschiedlichen Löslichkeitseigenschaften der Faser-Komponente und der Matrix-Komponente des mindestens einen aufgewickelten Mehrkomponentenfadens ausgenutzt. Das Polymerrohr wird mit einem Lösungsmittel oder einem Lösungsmittelgemisch versetzt, wodurch die Matrix-Komponente des mindestens einen aufgewickelten Mehrkomponentenfadens teilweise gelöst oder vollständig gelöst oder angelöst wird, ohne dass die Faser-Komponente in Lösung gebracht wird. Anschließend wird das Lösungsmittel vorzugsweise durch Verdunsten unter Vakuum entfernt. Dabei verfestigt sich die Matrix-Komponente so, dass ein porenfreies massives Polymerrohr erhalten wird. Das Versetzen mit einem Lösungsmittel oder einem Lösungsmittelgemisch und anschließendem Entfernen des Lösungsmittels kann einmal oder mehrmals durchgeführt werden, falls die gewünschte Homogenität des porenfreien Polymerrohrs nach einer Durchführung noch nicht erreicht ist. Bevorzugt ist, wenn die Lösungsmittel für das lösungsmittelbasierte Vereinigen des mindestens einen gerichtet aufgewickelten Mehrkomponentenfadens zu einem porenfreien Polymerrohr flüchtig sind und einen Siedepunkt unterhalb 80°C besitzen. Besonders bevorzugt sind Trifluorethanol, Chloroform, Dichlormethan und Hexafluorisopropanol als Lösungsmittel für das lösungsmittelbasierte Vereinigen. Somit ist erfindungsgemäß für das lösungsmittelbasierte Vereinigen des mindestens einen gerichtet aufgewickelten Mehrkomponentenfadens zu einem porenfreien Polymerrohr bevorzugt, wenn die Matrix-Komponente nicht jedoch die Faser-Komponente unter Einwirkung von Trifluorethanol oder Chloroform oder Dichlormethan oder Hexafluorisopropanol oder eines Gemisches aus diesen Lösungsmitteln plastisch verformbar ist.

In einer Ausführungsform der vorliegenden Erfindung wird als Lösungsmittel für das Vereinigen des mindestens einen aufgewickelten Mehrkomponentenfadens zu einem porenfreien Polymerrohr Trifluorethanol verwendet, wobei die Faser-Komponente aus PLLA und die Matrix-Komponente aus Poly(ε-caprolacton) besteht.

In einer weiteren Ausführungsform der vorliegenden Erfindung wird als Lösungsmittel für das Vereinigen des mindestens einen aufgewickelten Mehrkomponentenfadens zu einem porenfreien Polymerrohr Chloroform verwendet, wobei die Faser-Komponente aus Polyglycolsäure und die Matrix-Komponente aus Poly(ε-caprolacton) besteht.
Somit betrifft die vorliegende Erfindung auch Verfahren zur Herstellung eines Polymerrohrs, bevorzugt eines (bio)resorbierbaren Polymerrohrs, umfassend die folgenden Schritte:
Aufwicklung mindestens eines Mehrkomponentenfadens mit einem Durchmesser ≤ 200 µm, der aus einer Faser-Komponente und einer Matrix-Komponente besteht, zu einem Polymerrohr mit anschließender plastischer Verformung der Matrix-Komponente unter chemischen und/oder physikalischen Bedingungen unten denen die Faser-Komponente nicht plastisch verformbar ist zwecks Herstellung eines porenfreien Polymerrohrs und laserschneiden eines Stents aus dem porenfreien Polymerrohr.

Zudem betrifft die vorliegende Erfindung Stents erhältlich gemäß dem obigen Herstellungsverfahren.

Bei dem schmelzbasierten Vereinigen oder Verschmelzen werden die unterschiedlichen Schmelzpunkte der Faser-Komponente und der Matrix-Komponente des mindestens einen aufgewickelten Mehrkomponentenfadens genutzt. Vorzugsweise kann das Verschmelzen des mindestens einen aufgewickelten Mehrkomponentenfadens in einer Vakuumkammer unter Vakuum erfolgen. Durch das Erzeugen des Vakuums wird die Luft aus den noch vorhandenen minimalen Zwischenräumen der eng beieinander liegenden Mehrkomponentenfäden eliminiert und die durch das Verschmelzen erzeugten Polymerrohre sind besonders porenfrei, so wie durch Extrusion oder Spritzgießen hergestellte Polymerrohre. Das Verschmelzen der aufgewickelten Mehrkomponentenfäden und vorzugsweise der gerichtet aufgewickelten Mehrkomponentenfäden erfolgt vorzugsweise unterhalb des Schmelzbereichs der Faser-Komponente und oberhalb des Schmelzbereichs der Matrix-Komponente. Es ist bevorzugt, dass das Verschmelzen mindestens 10 °C unterhalb des Schmelzbereichs der Faser-Komponente, weiter bevorzugt mindestens 15 °C und am meisten bevorzugt mindestens 20 °C unterhalb des Schmelzpunktes oder des Schmelzbereichs der Faser-Komponente durchgeführt wird, so dass die Ausrichtung der Molekülketten in der Faser-Komponente erhalten bleibt. Die Molekülketten oder synonym als Polymerketten bezeichnet sind in der Polymerfaser oder den Polymerfasern der Faser-Komponente entlang der Längsachse des Mehrkomponentenfadens ausgerichtet. Beim Verschmelzen können unterschiedliche Temperaturprofile erzeugt werden, die beispielsweise sehr hohe Temperatur-Gradienten oder Haltezeiten einschließen können. Es ist bevorzugt, dass die Verschmelzung durch eine Zwangskühlung beendet wird.

Somit betrifft die vorliegende Erfindung ein weiteres bevorzugtes Verfahren zur Herstellung eines porenfreien Polymerrohrs und bevorzugt eines bioresorbierbaren porenfreien Polymerrohrs umfassend den folgenden Schritt:
porenfreies Vereinigen des mindestens einen aufgewickelten Mehrkomponentenfadens, wobei der aufgewickelte mindestens eine Mehrkomponentenfaden unter Beibehaltung der Ausrichtung der Molekülketten entlang der Fadenlängsachse porenfrei vereinigt wird.

Ein weiterer Aspekt der vorliegenden Erfindung betrifft ein Verfahren zur Herstellung eines porenfreien Polymerrohrs und bevorzugt eines bioresorbierbaren porenfreien Polymerrohrs umfassend den folgenden Schritt:
porenfreies Vereinigen des mindestens einen aufgewickelten Mehrkomponentenfadens im Vakuum.

Ebenso betrifft die vorliegende Erfindung ein Verfahren zur Herstellung eines porenfreien Polymerrohrs und bevorzugt eines bioresorbierbaren porenfreien Polymerrohrs umfassend den folgenden Schritt:
porenfreies Vereinigen des mindestens einen aufgewickelten Mehrkomponentenfadens, wobei der mindestens eine aufgewickelte Mehrkomponentenfaden lösungsmittelbasiert oder durch Schmelzen porenfrei vereinigt wird.

Ein weiterer Aspekt der vorliegenden Erfindung betrifft ein weiteres bevorzugtes Verfahren zur Herstellung eines porenfreien Polymerrohrs und bevorzugt eines bioresorbierbaren porenfreien Polymerrohrs umfassend die folgenden Schritte:
a) Aufwickeln oder gerichtetes Aufwickeln mindestens eines Mehrkomponentenfadens mit einem Durchmesser von ≤ 200 µm vorzugsweise auf einen sich drehenden Dorn;
b) porenfreies Vereinigen des mindestens einen aufgewickelten Mehrkomponentenfadens, wobei der aufgewickelte mindestens eine Mehrkomponentenfaden unter Beibehaltung der Ausrichtung der Molekülketten entlang der Fadenlängsachse porenfrei vereinigt wird.

Ein weiterer Aspekt der vorliegenden Erfindung betrifft ein Verfahren zur Herstellung eines porenfreien Polymerrohrs und bevorzugt eines bioresorbierbaren porenfreien Polymerrohrs umfassend die folgenden Schritte:
a) Aufwickeln oder gerichtetes Aufwickeln mindestens eines Mehrkomponentenfadens mit einem Durchmesser von ≤ 200 µm vorzugsweise auf einen sich drehenden Dorn zu einem Polymerrohr;
b) porenfreies Vereinigen des mindestens einen aufgewickelten Mehrkomponentenfadens, wobei der mindestens eine aufgewickelte Mehrkomponentenfaden lösungsmittelbasiert oder durch Schmelzen zu einem porenfreien Polymerrohr vereinigt wird.

Ein weiterer Aspekt der vorliegenden Erfindung betrifft ein Verfahren zur Herstellung eines porenfreien Polymerrohrs und bevorzugt eines bioresorbierbaren porenfreien Polymerrohrs umfassend die folgenden Schritte:
a) Aufwickeln oder gerichtetes Aufwickeln mindestens eines Mehrkomponentenfadens mit einem Durchmesser von ≤ 200 µm vorzugsweise auf einen sich drehenden Dorn;
b) porenfreies Vereinigen des mindestens einen aufgewickelten Mehrkomponentenfadens durch Verschmelzen im Vakuum bei einer Temperatur von mindestens 10°C unterhalb des Schmelzbereichs der Faser-Komponente und mindestens 10°C oberhalb des Schmelzbereichs der Matrix-Komponente des aufgewickelten Mehrkomponentenfadens.

Damit liegen die erfindungsgemäß hergestellten porenfreien Polymerrohre bereits in kristalliner oder teilkristalliner Form vor, ohne dass ein Verstrecken erforderlich ist. Die Kristallinität wird sowohl durch Ausrichtung der Moleküle in den Mehrkomponentenfäden als auch durch die gerichtete Aufwicklung des Mehrkomponentenfadens / der Mehrkomponentenfäden erzeugt. Eine bevorzugte Ausführungsform umfasst daher ein Polymerrohr hergestellt aus mindestens einem aufgewickelten Mehrkomponentenfaden, wobei der aus mindestens eine aufgewickelte Mehrkomponentenfaden einen Durchmesser im Bereich von 50 nm bis 200 µm aufweist und die einzelnen Wicklungen des Mehrkomponentenfadens, zumindest in vordefinierten Bereichen, parallel zueinander liegen. Diese Bereiche können z.B. einzelne Schichten von Wicklungen sein, aber auch Bereiche des Polymerrohrs aus denen später eine Strebe eines Stents entsteht und in denen die Richtung des Fadens bzw. der Winkel der Wicklung der späteren Beanspruchung der Strebe entspricht.

Das thermische Verschmelzen der gewickelten Mehrkomponentenfäden kann erfindungsgemäß wie folgt durchgeführt werden:
Die in Schritt a) aufgewickelten Polymerfäden können noch auf dem Dorn unter erhöhter Temperatur und additional unter Druck von außen zur Schließung der Poren verschmolzen werden. Der Druck kann z.B. durch eine außen anliegende Walze erzeugt werden.

Eine andere Methode ist das Aufdehnen des in Schritt a) aufgewickelten Polymerrohres unter Temperatur und Druck von innen, wobei neben dem Verschmelzen die Fäden gleichzeitig gereckt werden. Dieses Verfahren ist der Ballonherstellung (dem Ballonblasen) bei der Ballonkatheterherstellung ähnlich.

Es konnte überraschend gezeigt werden, dass die Zugfestigkeiten eines erfindungsgemäß hergestellten porenfreien Polymerrohrs eine hohe Richtungsabhängigkeit zeigen. Es wird vermutet, dass dies auf die unidirektional ausgerichteten Polymerketten oder Molekülketten der Polymerfaser oder Polymerfasern zurückzuführen ist. Besonders vorteilhaft ist, dass zudem die Bruchdehnung in Richtung der Molekülketten trotz der hohen Zugfestigkeit mit über 100 % bei den erfindungsgemäßen Polymerrohren sehr hoch ist, d.h. die Verspannungen (innere Spannungen) des Materials sind gering. Dies ist ein grundsätzlicher, entscheidender Vorteil zu den durch Verstrecken verfestigten Rohren aus dem Stand der Technik, bei denen die Bruchdehnung für hohe Zugfestigkeiten vermindert ist.

Dennoch lässt sich auch bei einem erfindungsgemäß hergestellten Polymerrohr oder dem daraus hergestellten porenfreien Polymerrohr die erhöhte Festigkeit durch Verstrecken, d.h. Dehnen bzw. Verfestigen des Polymerrohrs weiter steigern. Bevorzugt sind dabei folgende Vorgehensweisen:
- Bereits beim Aufwickeln auf den Dorn können die Polymerfäden (mit den bereits ausgerichteten Molekülketten) gereckt werden.
- Eine Verfestigung kann durch Aufdehnen der gewickelten, aber noch nicht vereinten Rohre erfolgen.
- Eine additionale Kombination aus beiden Verfahren (Recken des Fadens beim Aufwickeln und zusätzlich anschließendes Verstrecken des Rohres) ist möglich.
- Es ist möglich, das Vereinigen der Fäden mit dem Verstrecken des gewickelten Rohres in einem thermomechanischen Vorgang zu kombinieren.
- Das fertig hergestellte Rohr (gewickelt und vereinigt) lässt sich durch einen anschließenden Dehnvorgang verstrecken.

Ein bevorzugtes, erfindungsgemäßes Verfahren zur Herstellung eines Polymerrohrs umfasst somit, dass der mindestens eine Mehrkomponentenfaden beim Aufwickeln auf den sich drehenden Dorn gereckt und damit verfestigt wird.

Ein weiteres bevorzugtes, erfindungsgemäßes Verfahren zur Herstellung eines porenfreien Polymerrohrs ergibt sich dadurch, dass das Polymerrohr vor, während oder nach der Vereinigung auf einen Nenndurchmesser aufgedehnt und dabei verfestigt wird.

Daher ergeben sich die folgenden im Sinne der Erfindung bevorzugten Verfahren zur Herstellung eines porenfreien Polymerrohrs:
Verfahren zur Herstellung eines porenfreien Polymerrohrs und bevorzugt eines bioresorbierbaren porenfreien Polymerrohrs, welches die folgenden Schritte umfasst:
   a) Aufwickeln oder gerichtetes Aufwickeln mindestens eines Mehrkomponentenfadens mit einem Durchmesser von ≤ 200 µm auf einen sich drehenden Dorn zu einem Polymerrohr; und beim Aufwickeln recken des mindestens einen Mehrkomponentenfadens
   b) porenfreies Vereinigen des mindestens einen aufgewickelten Mehrkomponentenfadens.

Beim Recken setzt man beispielsweise die Mehrkomponentenfäden zum Verformen unter Zugspannung, so dass sich die ungeordneten Polymere und die teilkristallinen Bereiche parallel zur Zugrichtung ausrichten.

Verfahren zur Herstellung eines porenfreien Polymerrohrs und bevorzugt eines bioresorbierbaren porenfreien Polymerrohrs, welches die folgenden Schritte umfasst:
a) Aufwickeln mindestens eines Mehrkomponentenfadens mit einem Durchmesser von ≤ 200 µm zu einem Polymerrohr;
a') Verfestigung durch Aufdehnen des Polymerrohrs;
b) Vereinigen des mindestens einen aufgewickelten Mehrkomponentenfadens.

Verfahren zur Herstellung eines porenfreien Polymerrohrs und bevorzugt eines bioresorbierbaren porenfreien Polymerrohrs, welches die folgenden Schritte umfasst:
a) Aufwickeln oder gerichtetes Aufwickeln mindestens eines Mehrkomponentenfadens mit einem Durchmesser von ≤ 200 µm auf einen sich drehenden Dorn zu einem Polymerrohr; und beim Aufwickeln Recken des mindestens einen Mehrkomponentenfadens;
a') Verfestigung durch Aufdehnen des Polymerrohrs;
b) porenfreies Vereinigen des mindestens einen aufgewickelten Mehrkomponentenfadens.

Verfahren zur Herstellung eines porenfreien Polymerrohrs und bevorzugt eines bioresorbierbaren porenfreien Polymerrohrs, welches die folgenden Schritte umfasst:
a) Aufwickeln oder gerichtetes Aufwickeln mindestens eines Mehrkomponentenfadens mit einem Durchmesser von ≤ 200 µm zu einem Polymerrohr;
b) porenfreies Vereinigen des mindestens einen aufgewickelten Mehrkomponentenfadens und parallel zum Vereinigen Verstrecken des Polymerrohrs.

Verfahren zur Herstellung eines porenfreien Polymerrohrs und bevorzugt eines bioresorbierbaren porenfreien Polymerrohrs, welches die folgenden Schritte umfasst:
a) Aufwickeln oder gerichtetes Aufwickeln mindestens eines Mehrkomponentenfadens mit einem Durchmesser von ≤ 200 µm zu einem Polymerrohr;
b) porenfreies Vereinigen des mindestens einen aufgewickelten Mehrkomponentenfadens;
b') Verstrecken des vereinigten Polymerrohrs durch Dehnung.

Das Verstrecken des erfindungsgemäß hergestellten Polymerrohrs kann bei Raumtemperatur vorzugsweise aber bei erhöhter Temperatur erfolgen.

Zur Einstellung und/oder Fixierung der mechanischen Eigenschaften der erfindungsgemäßen Polymerrohre, insbesondere wenn ein Verstrecken stattgefunden hat, kann noch eine thermische Nachbehandlung durchgeführt werden. Durch Tempern bei Temperaturen unterhalb des Schmelzpunktes wird die Kristallinität erhöht. Durch Abschrecken können diese kristallinen Strukturen, aber auch die amorphe Strukturen fixiert werden.

Überraschend wurde herausgefunden, dass Polymerrohre, hergestellt durch Aufwicklung möglichst dünner Mehrkomponentenfäden, z.B. auf sich drehenden Dornen, eine erhöhte Festigkeit bei gleichzeitig optimaler Bruchdehnung aufweisen. In den dünnen Fäden mit Durchmessern im Mikrometerbereich oder Submikrometerbereich (mit einem Durchmesser im Bereich von 50 nm bis 200 µm) sind die Moleküle bereits weitgehend unidirektional ausgerichtet.

Wie erläutert, führen die derzeitigen festigkeitssteigernden Methoden, angewendet bei Polymerrohren aus dem Stand der Technik, zu starken Einschränkungen bei der Verwendung als Ausgangsmaterial für Polymerstents. Die erfindungsgemäße Herstellung von Polymerrohren beseitigt die starken Einschränkungen bzw. verbessert die Eigenschaften der Rohre, was insbesondere für deren Verwendung zur Herstellung von Stents und anderen Gefäßimplantaten entscheidend ist.

Beim Verstrecken (Dehnen) von Polymerrohren und porenfreien Polymerrohren laufen grundsätzlich zwei Effekte parallel ab:
A) Die einzelnen langkettigen Moleküle werden in Dehnungsrichtung entknäult und gestreckt. In Richtung der Molekülketten ist die Festigkeit erhöht und quer zur Molekülrichtung geringer.
B) Beim Dehnen bewegen sich die Molekülketten aber auch zueinander, gleiten aufeinander ab und werden zueinander verspannt (Verfestigung). Es entstehen innere Spannungen. Mit zunehmenden inneren Spannungen nimmt die Dehnbarkeit des Werkstoffes ab.

Das Verstrecken ist daher nur begrenzt möglich, da Polymerrohre nur begrenzt ohne Bruchinduzierung gedehnt werden können. Zum Einen lassen sich die Moleküle nicht beliebig strecken und zum Anderen dürfen die inneren Spannungen die Bruchfestigkeit nicht überschreiten.

Erfindungsgemäß sollen die Vorgänge der Molekülausrichtung und der Verfestigung während der erfindungsgemäßen Herstellung der Polymerrohre völlig, zumindest aber weitgehend, getrennt voneinander ablaufen, um für beide Vorgänge ein Optimum zu erreichen.

Die Molekülausrichtung wird schon beim ersten Schritt der Herstellung der Polymerrohre, nämlich beim Aufwickeln der Mehrkomponentenfäden gewährleistet. Die Rohrherstellung erfolgt durch Aufwicklung möglichst dünner Mehrkomponentenfäden, bevorzugt auf drehenden Dornen oder Wellen oder Sulen. In den dünnen Fäden mit Durchmessern im Mikrometerbereich (≤ 200 µm) oder Submikrometerbereich (≤ 1 µm) sind die Moleküle bereits weitgehend unidirektional ausgerichtet. Die Mehrkomponentenfadenherstellung kann z.B. durch Extrusion aus kleinsten Spinndüsen (Schmelzspinnen) erfolgen oder vorzugsweise durch Lösungsspinnen. Eine Dehnung oder Verfestigung findet danach in einem oder mehreren weiteren Schritten statt.

Ein weiterer Aspekt der vorliegenden Erfindung betrifft lasergeschnittene Stents aus Polymerrohren bestehend aus gerichtet aufgewickelten Mehrkomponentenfäden, welche nicht miteinander porenfrei vereinigt sind. Durch den Laserschnitt werden die geschnittenen Mehrkomponentenfäden an der Schnittkante miteinander verschmolzen, so dass keine einzelnen Fäden sich aus dem fertig geschnittenen Stent ablösen können, aber aufgrund der erhaltenen Fadenstruktur der Stent vorteilhafte Eigenschaften hinsichtlich Zugfestigkeit und Rückstellkraft in Richtung des Nominaldurchmessers aufweist.

Die starke Richtungsabhängigkeit der Festigkeit der erfindungsgemäß hergestellten Rohre kann man insbesondere für die Herstellung von Gefäßimplantaten, vorzugsweise **Stents** und insbesondere bevorzugt bioresorbierbaren Stents nutzen. Unter Stents werden hierbei gitterförmige oder netzförmige Endoprothesen verstanden, die in ein Hohlorgan oder einen Körperhohlraum eingesetzt werden, um diesen offen zu halten. Das Grundgerüst eines Stents, womit hier die polymeren Streben ohne Beschichtung bezeichnet werden, bildet kein massives Rohr, sondern ein expandierbares Gittergeflecht. Betrachtet man beispielsweise das Grundgerüst eines vaskulären Stents, so wird dieses aus einem massiven Rohr z.B. mittels Laser geschnitten, so dass sich einzelne, möglichst dünne Streben ergeben, welche untereinander verbunden sind. Die Anordnung und Ausformung der Streben und Knotenpunkte wird als Stentdesign bezeichnet. Im Sinne der vorliegenden Erfindung können alle üblichen Stentgeometrien verwendet werden.

Stents oder Gefäßstützen benötigen eine hohe radiale Festigkeit, um das Gefäß nach der Dilatation (Aufdehnung) offen zu halten. Hingegen ist in axialer Richtung, d.h. in Längsrichtung der Stents keine hohe Festigkeit gefordert. In radialer Richtung (Umfangsrichtung) ist zudem eine hohe Bruchdehnung gefordert, um den Stent bei Implantation mittels Ballonkatheter ausreichend und ohne Bruchgefahr dehnen zu können. Die erfindungsgemäß hergestellten porenfreien Polymerrohre und Polymerrohre weisen genau diese Eigenschaften auf. Sie zeichnen sich durch eine hohe Festigkeit bei gleichzeitiger, hoher Bruchdehnung in radialer Richtung, d.h. in Umfangsrichtung, aus.

Ein bevorzugter Aspekt der vorliegenden Erfindung ist es daher Polymerrohre zur Verfügung zu stellen, die zur Herstellung eines Stents besonders gut geeignet sind. Diese Polymerrohre, vorzugsweise resorbierbaren Polymerrohre, können nach einem der hierin beschriebenen, erfindungsgemäßen Verfahren hergestellt werden.

Des Weiteren umfasst die vorliegende Erfindung daher einen Stent oder ein Gefäßimplantat, bevorzugt einen resorbierbaren Stent, hergestellt aus einem der hierin offenbarten Polymerrohre. Es handelt sich bei dem resorbierbaren Stent bevorzugt um einen Stent für Blutgefäße, Harnwege, Atemwege, Gallenwege oder den Verdauungstrakt. Unter diesen Stents sind wiederum die Stents für Blutgefäße oder allgemeiner für das Herz-Kreislaufsystem bevorzugt.

Somit betrifft ein weiterer Aspekt der vorliegenden Erfindung Stents erhältlich durch Vereinigen eines Polymerrohrs bestehend aus mindestens einem aufgewickelten Mehrkomponentenfaden mit einem Durchmesser ≤ 200 µm, wobei der mindestens eine aufgewickelte Mehrkomponentenfaden aus einer Faser-Komponente und einer Matrix-Komponente besteht

Zudem betrifft die vorliegende Erfindung Stents bestehend aus einer Faser-Komponente und einer Polymermatrix, wobei die Faser-Komponente ein geringes radiales Profil aufweist, und wobei das Gittergeflecht porenfrei ist.

Beim Schneiden eines Stents werden Flächen zwischen den einzelnen Streben herausgeschnitten. Ein Stent oder ein vaskuläres Implantat weist daher eine Vielzahl von massiven Gerüstkomponenten (z.B. Streben in Form von Ringen, Spiralen, Wellen und Drähten) auf, sowie eine Vielzahl von Zwischenräumen zwischen diesen massiven Komponenten. Bei der gängigen Ausführungsform von Endoprothesen oder Stents laufen die Streben in Knotenpunkten zusammen. Es gibt jedoch auch Ausführungsformen von Endoprothesen, bei denen keine oder fast keine Knotenpunkte vorhanden sind und die Streben z.B. die Form von Ringen oder Spiralen haben. Bevorzugt handelt es sich um ballonexpandierbare Stents, welche mittels Katheter bis zur erkrankten oder zu behandelnden Stelle geschoben werden, wo die Stents auf ihren definierten Normdurchmesser aufgeweitet werden.

Ferner ist ein Kriechen des Materials, d.h. der gecrimpten bzw. im Gefäß dilatierten Stents unerwünscht, was durch eine Begrenzung der Dehnung bzw. der Verstreckung erzielt werden kann. Um unerwünschte Kriechvorgänge der implantierten Stents weitgehend zu vermeiden, hat es sich erfindungsgemäß als vorteilhaft erwiesen, Stents z.B. durch Laserschneiden im gedehnten Zustand herzustellen. Das bedeutet, die Stents werden aus erfindungsgemäßen Polymerrohren hergestellt, deren Durchmesser dem Nenndurchmesser, also dem Innendurchmesser des Stents im aufgeweiteten Zustand, entspricht. Danach kann der erfindungsgemäße Stent dennoch ohne Schwierigkeiten auf einen Katheterballon aufgebracht (gecrimpt) werden. Die Erfindung betrifft damit Stents hergestellt aus den erfindungsgemäßen Polymerrohren, wobei der Innendurchmesser des Polymerrohrs (des nicht aufgedehnten Polymerrohrs) dem Innendurchmesser des Stents nach Implantation entspricht.

Die erfindungsgemäßen Polymerrohre zur Herstellung eines Stents weisen daher vorzugsweise Innendurchmesser auf, die den aufgedehnten Stents entsprechen. Es ist daher bevorzugt, wenn das erfindungsgemäße Polymerrohr einen Innendurchmesser von 0,5 - 15 mm, weiter bevorzugt von 1 - 10 mm und noch weiter bevorzugt von 1,5 - 6 mm aufweist. Aus Polymerrohren dieser Durchmesser, die den Durchmessern dilatierter Stents entsprechen (Nenndurchmesser oder Nominaldurchmesser), werden die Gefäßimplantate bevorzugt lasergeschnitten. Damit liegen die hergestellten Stents im aufgedehnten (dilatierten) Zustand vor.

Außerdem ist dies vorteilhaft, da im aufgedehnten Zustand die Stentstege oder Stentstreben vorzugsweise in Umfangsrichtung weisen und damit in Richtung der aufgewickelten Polymerfäden respektive in Richtung der Molekülketten bzw. der hohen Festigkeiten. Anschließend werden die Stents auf den Ballonkatheter gecrimpt, um sie implantieren zu können.

Die vorliegende Erfindung betrifft somit Verfahren zur Herstellung eines porenfreien Stents, bevorzugt eines resorbierbaren porenfreien Stents, umfassend die folgenden Schritte:
a) Aufwickeln oder gerichtetes Aufwickeln mindestens eines aus einer Faser-Komponente und einer Matrix-Komponente bestehenden Mehrkomponentenfadens mit einem Durchmesser von ≤ 200 µm zu einem Polymerrohr;
b) porenfreies Vereinigen des mindestens einen aufgewickelten Mehrkomponentenfadens; und
c) Schneiden eines Stents aus dem porenfreien Polymerrohr, vorzugsweise mittels Laser.

Ein bevorzugtes Verfahren der vorliegenden Erfindung ist ein Verfahren zur Herstellung eines porenfreien Stents, bevorzugt eines resorbierbaren porenfreien Stents, umfassend die folgenden Schritte:
a) Aufwickeln oder gerichtetes Aufwickeln mindestens eines aus einer Faser-Komponente und einer Matrix-Komponente bestehenden Mehrkomponentenfadens mit einem Durchmesser von ≤ 200 µm zu einem Polymerrohr;
b) porenfreies Vereinigen des mindestens einen aufgewickelten Mehrkomponentenfadens; und
c) Schneiden eines Stents aus dem porenfreien Polymerrohr, vorzugsweise mittels Laser, wobei der Durchmesser des porenfreien Polymerrohrs dem Nenndurchmesser des Stents entspricht.

Die vorliegende Erfindung ist zudem auf ein Verfahren zur Herstellung eines porenfreien Stents und vorzugsweise eines bioresorbierbaren porenfreien Stents gerichtet, umfassend die folgenden Schritte:
a) Aufwickeln oder gerichtetes Aufwickeln mindestens eines aus einer Faser-Komponente und einer Matrix-Komponente bestehenden Mehrkomponentenfadens mit einem Durchmesser von ≤ 200 µm vorzugsweise auf einen sich drehenden Dorn;
b) porenfreies Vereinigen des mindestens einen aufgewickelten Mehrkomponentenfadens, wobei der aufgewickelte mindestens eine Mehrkomponentenfaden unter Beibehaltung der Ausrichtung der Polymerketten oder Molekülketten der Polymerfaser oder Polymerfasern entlang der Fadenlängsachse porenfrei vereinigt wird; und
c) Schneiden eines Stents aus dem porenfreien Polymerrohr, vorzugsweise mittels Laser.

Ein weiterer Aspekt der vorliegenden Erfindung betrifft ein Verfahren zur Herstellung eines porenfreien Stents und vorzugsweise eines bioresorbierbaren porenfreien Stents umfassend die folgenden Schritte:
a) Aufwickeln oder gerichtetes Aufwickeln mindestens eines aus einer Faser-Komponente und einer Matrix-Komponente bestehenden Mehrkomponentenfadens mit einem Durchmesser von ≤ 200 µm vorzugsweise auf einen sich drehenden Dorn zu einem Polymerrohr;
b) porenfreies Vereinigen des mindestens einen aufgewickelten Mehrkomponentenfadens im Vakuum; und
c) Schneiden eines Stents aus dem porenfreien Polymerrohr, vorzugsweise mittels Laser.

Ein weiterer Aspekt der vorliegenden Erfindung betrifft ein Verfahren zur Herstellung eines porenfreien Stents und vorzugsweise eines bioresorbierbaren porenfreien Stents umfassend die folgenden Schritte:
a) Aufwickeln oder gerichtetes Aufwickeln mindestens eines aus einer Faser-Komponente und einer Matrix-Komponente bestehenden Mehrkomponentenfadens mit einem Durchmesser von ≤ 200 µm vorzugsweise auf einen sich drehenden Dorn;
b) porenfreies Vereinigen des mindestens einen aufgewickelten Mehrkomponentenfadens durch Verschmelzen im Vakuum bei einer Temperatur von mindestens 10°C unterhalb des Schmelzbereichs der Faser-Komponente und mindestens 10°C oberhalb des Schmelzbereichs der Matrix-Komponente des aufgewickelten Mehrkomponentenfadens; und
c) Schneiden eines Stents aus dem porenfreien Polymerrohr, vorzugsweise mittels Laser.

Ein weiterer Aspekt der vorliegenden Erfindung betrifft ein Verfahren zur Herstellung eines porenfreien Stents und vorzugsweise eines bioresorbierbaren porenfreien Stents umfassend die folgenden Schritte:
a) Aufwickeln oder gerichtetes Aufwickeln mindestens eines aus einer Faser-Komponente und einer Matrix-Komponente bestehenden Mehrkomponentenfadens mit einem Durchmesser von ≤ 200 µm vorzugsweise auf einen sich drehenden Dorn zu einem Polymerrohr;
b) plastische Verformung der Matrix-Komponente unter chemischen und/oder physikalischen Bedingungen unter denen die Faser-Komponente nicht plastisch verformbar ist; und
c) Schneiden eines Stents aus dem porenfreien Polymerrohr, vorzugsweise mittels Laser.

Bei allen vorgenannten Verfahren verläuft die Faser-Komponente entlang der Längsachse des Mehrkomponentenfadens und die Matrix-Komponente umgibt die Faser-Komponente. Dies ist in Figur 1a gezeigt, wo die Matrix-Komponente die Faser-Komponente konzentrisch umgibt wie eine Isolierschicht einen Draht umgibt. In Figur 1b besteht die Faser-Komponente hingegen aus einer Vielzahl an Einzelfasern, welche in die Matrix-Komponente eingebettet sind und daher auch von der Matrix-Komponente umgeben werden.

Somit betrifft die vorliegende Erfindung auch einen Stent bestehend aus einem plastisch verformten lasergeschnittenen porenfreien Polymerrohr, wobei das Polymerrohr aus mindestens einem gerichtet aufgewickelten Mehrkomponentenfaden mit einem Durchmesser ≤ 200 µm besteht und der mindestens eine gerichtet aufgewickelte Mehrkomponentenfaden aus einer Faser-Komponente und einer Matrix-Komponente besteht und die Faser-Komponente entlang der Längsachse des mindestens einen Mehrkomponentenfadens verläuft und die Matrix-Komponente die Faser-Komponente umgibt.

Derartige erfindungsgemäße Stents besitzen eine radiale Rückstellkraft in Richtung des Durchmessers des Polymerrohrs aus dem sie hergestellt wurden. Ferner besitzen sie eine hohe radiale Festigkeit. Erfindungsgemäß sind somit Stents bestehend aus einem lasergeschnittenen Polymerrohr wie hierin offenbart.

Dabei treffen alle hierin gemachten Aussagen zu bevorzugten Ausführungsformen eines erfindungsgemäßen Verfahrens zur Herstellung eines Polymerrohrs entsprechend auch auf die Verfahren zur Herstellung des Stents zu.

Dieses Verfahren der Stentherstellung im gedehnten Zustand weist den weiteren Vorteil auf, dass unerwünschte Kriechvorgänge der Polymere weitgehend vermieden werden. Verformte Polymere neigen zum Kriechen in ihre Ursprungsform. Ein Stent, der im ungedehnten (gecrimpten) Zustand hergestellt und anschließend gedehnt wird, neigt zum Kriechen in Richtung seines ursprünglichen, kleineren Durchmessers. Dies ist für eine Stentimplantation nachteilig, da der implantierte Stent seinen Durchmesser im Gefäß wieder verringert. Dieses Verhalten wird bei den derzeitig zugelassenen, bioresorbierbaren Stents beobachtet.

Wird der Stent hingegen aus einem Polymerrohr mit einem großen Rohrdurchmesser, der dem dilatierten Gefäßdurchmesser entspricht, geschnitten, so wird er diesen Durchmesser nach der Dilatation im Gefäß beibehalten. Es ist erfindungsgemäß bevorzugt, wenn der Stent aus einem erfindungsgemäßen Polymerrohr mittels Laser ausgeschnitten wird. Dabei ist es weiter bevorzugt, wenn das erfindungsgemäße Polymerrohr dabei einen Durchmesser aufweist, welcher dem Durchmesser des aufgedehnten (bzw. aufgeweiteten oder inflatierten) Stents entspricht. Dabei entspricht der Innendurchmesser des aufgedehnten Stents dem Innendurchmesser des erfindungsgemäßen Polymerrohrs, welches zu seiner Herstellung verwendet wird. Das gleiche gilt für die Außendurchmesser, es sei denn der Stent wird nach der Herstellung aus dem Polymerrohr noch mit einer Beschichtung, z.B. einer wirkstofffreisetzenden Beschichtung, versehen.

Ein weiterer Vorteil der Herstellung der Stents aus Rohren deren Durchmesser dem aufgedehnten Durchmesser, d.h. Nenndurchmesser, des Stents entspricht ist, dass die Bruchgefahr bei einem Überdehnen über den Nenndurchmesser unkritischer ist als bei Stents, die aus kleineren Rohrdurchmessern (zumeist dem Durchmesser der gecrimpten, deflatierten Stents entsprechend) hergestellt wurden. Da die Festigkeit der Polymerrohre aus dem Stand der Technik zu Lasten der Dehnbarkeit optimiert wurde, sind die aus kleineren Rohrdurchmessern hergestellten Stents nur sehr begrenzt dehnbar und eine Überdehnung führt schnell zum Bruch. Bei den erfindungsgemäß hergestellten Stents sind bei gleicher, begrenzter Dehnbarkeit größere Gefäßdurchmesser erreichbar.

Es ist zudem möglich, die erfindungsgemäß hergestellten Polymerrohre noch zielgerichteter an die mechanischen Anforderungen von Gefäßimplantaten anzupassen:
Beim Aufwickeln des Mehrkomponentenfadens wird ein sich drehender Dorn so hinund her bewegt, dass der Faden z.B. kreuzweise unter einem bestimmten Winkel aufgewickelt wird. Das kreuzweise oder lagenweise Aufwickeln einer oder mehrerer Mehrkomponentenfäden kann insbesondere beim Elektrospinnen auch durch die Ablenkung der Fasern in einem elektrischen Feld erfolgen.

Das gerichtete Aufwickeln kann gezielt im Hinblick auf die späteren Belastungen (Richtungen der Spannungsbeanspruchungen) der einzelnen Stege oder Streben des Stents nach Implantation, z.B. in ein Blutgefäß, geschehen. Die Mehrkomponentenfäden sollen dabei vorzugsweise in Richtung der höchsten Festigkeitsbeanspruchung liegen. Ferner ist es möglich, den Mehrkomponentenfaden lagenweise d.h. in Schichten mit verschiedenen Winkeln aufzuwickeln und so gewünschte zielgerichtete Festigkeiten zu erreichen. Es ist auch möglich, in axialer Richtung des Rohres unterschiedliche Rohrwandstärken zu realisieren, die den unterschiedlichen Festigkeitsansprüchen eines Stents z.B. in der Mitte und an den Enden Rechnung trägt. Hierzu werden die Wicklungsstärken, d.h. die Anzahl der Wicklungen des Fadens in Längsrichtung auf dem Dorn variiert.

Ein anderes bevorzugtes Verfahren zur Herstellung eines Polymerrohrs gemäß der vorliegenden Erfindung, umfasst einen Schritt a) bei dem der Mehrkomponentenfaden zielgerichtet lagen- und kreuzweise unter einem definierten Winkel aufgewickelt wird.

Daher ergibt sich für die Herstellung eines porenfreien Polymerrohrs und bevorzugt eines bioresorbierbaren porenfreien Polymerrohrs ein bevorzugtes Verfahren, welches die folgenden Schritte umfasst:
a) Aufwickeln oder gerichtetes Aufwickeln mindestens eines Mehrkomponentenfadens mit einem Durchmesser von ≤ 200 µm auf einen sich drehenden Dorn zu einem Polymerrohr, wobei der Dorn dabei so hin und her bewegt wird, dass der Mehrkomponentenfaden unter einem bestimmten Winkel kreuzweise oder lagenweise aufgewickelt wird;
b) porenfreies Vereinigen des mindestens einen aufgewickelten Mehrkomponentenfadens.

Ein weiteres bevorzugtes Verfahren für die Herstellung eines porenfreien Polymerrohrs und bevorzugt eines bioresorbierbaren porenfreien Polymerrohrs umfasst die folgenden Schritte:
a) Aufwickeln oder gerichtetes Aufwickeln mindestens eines Mehrkomponentenfadens mit einem Durchmesser von ≤ 200 µm auf einen sich drehenden Dorn zu einem Polymerrohr, wobei der Dorn dabei so hin und her bewegt wird, dass der Mehrkomponentenfaden gemäß einem vorgegebenen Muster aufgewickelt wird;
b) porenfreies Vereinigen des mindestens einen aufgewickelten Mehrkomponentenfadens.

Dabei ist es bevorzugt, wenn das Muster entsprechend dem Stentdesign gewählt wird und z.B. spiralförmig ist.

Ein weiteres bevorzugtes Verfahren für die Herstellung eines porenfreien Polymerrohrs und bevorzugt eines bioresorbierbaren porenfreien Polymerrohrs umfasst die folgenden Schritte:
a) Aufwickeln oder gerichtetes Aufwickeln mindestens eines Mehrkomponentenfadens mit einem Durchmesser von ≤ 200 µm zu einem Polymerrohr, wobei der mindestens eine Mehrkomponentenfaden in mindestens zwei Schichten mit verschiedenen Winkeln aufgewickelt wird;
b) porenfreies Vereinigen des mindestens einen aufgewickelten Mehrkomponentenfadens.

Es ist dabei besonders bevorzugt, wenn die Winkel entsprechend der Festigkeitsbeanspruchung der aus den Rohren hergestellten Bauteile gewählt werden. Im Fall eines Stents sind dies vor allem Radialkräfte, die auf die einzelnen Stentstreben einwirken. Daher ist es von besonderem Vorteil, dass die Winkel je nach Stentdesign frei wählbar sind.

Es ist bevorzugt, wenn die Wicklungsstärken, d.h. die Anzahl der übereinanderliegenden Wicklungen bzw. Schichten des Mehrkomponentenfadens, bei den erfindungsgemäß hergestellten Polymerrohre zwischen 1 Wicklung und 1000 Wicklungen, weiter bevorzugt zwischen 2 Wicklungen und 600 Wicklungen, weiter bevorzugt zwischen 3 Wicklungen und 500 Wicklungen und noch weiter bevorzugt zwischen 4 Wicklungen und 300 Wicklungen liegt.

Wird der Faden wie beim Elektrospinnen direkt nach Bildung aufgewickelt kann er nicht auf konventionellem Wege gereckt werden. Die Aufwicklung dieser dünnen Mehrkomponentenfäden erfordert daher eine hohe Ablagegeschwindigkeiten, d.h. hohe Rotationsgeschwindigkeiten des aufwickelnden rotierenden Dorns. Wird der Faden unter einem Winkel z.B. kreuzweise aufgewickelt, so sind schnelle Hin- und Herbewegungen d.h. hohe axiale Verfahrgeschwindigkeiten des Dorns erforderlich.

Bei einem weiteren bevorzugten Verfahren zur Herstellung eines Polymerrohrs gemäß der vorliegenden Erfindung, werden beim Aufwickeln der Mehrkomponentenfäden **Wirkstoffe** eingebracht. Es handelt sich dabei bevorzugt um Wirkstoffe, die zur Verminderung einer Restenose verwendet werden können. Geeignete Wirkstoffe sind insbesondere antiproliferative oder antirestenotische Wirkstoffe.

Der mindestens eine eingesetzte antiproliferative oder antirestenotische Wirkstoff ist bevorzugt ausgewählt aus der Gruppe umfassend oder bestehend aus: Paclitaxel, Rapamycin und deren Derivaten, wie 6-α-Hydroxy-Paclitaxel, Baccatin, Docetaxel, Biolimus A9, Myolimus, Novolimus, Pimecrolimus, Tacroliums, Temsirolimus, Zotarolimus, Everolimus, Ridaforolimus oder weiteren sogenannten Taxanen, bzw "Limus"-Derivaten.

Es können auch Wirkstoffkombinationen eingebracht werden. Dabei können die Wirkstoffe in unterschiedlichen Konzentrationen vorliegen. Die Konzentration eines verwendeten Wirkstoffs kann ebenfalls zwischen verschiedenen Teilbereichen des erfindungsgemäßen Polymerrohrs und damit des daraus herstellbaren Stents variieren. So kann im Bereich der Enden des Polymerrohrs mehr Wirkstoff enthalten sein, als im mittleren Bereich. Alternativ kann auf der luminalen (blutseitigen) Rohrinnenseite ein anderer Wirkstoff eingebracht werden als auf der abluminalen (gefäßseitigen) Rohraußenseite. Denkbar wären ein Wirkstoff zur Förderung der Endothelialisierung auf der luminalen Rohrinnenseite und ein Wirkstoff zur Proliferationshemmung auf der abluminalen Rohraußenseite.

Beim lagenweisen Aufwickeln von lösungs- oder schmelzgesponnenen Mehrkomponentenfäden können Wirkstoffe zwischen den Mehrkomponentenfäden aufgebracht werden. Damit ist es möglich, die Wirkstoffe direkt in die Wand des Polymerrohrs zu integrieren. Der Mehrkomponentenfaden kann z.B. vor dem Aufwickeln mit einer Wirkstofflösung imprägniert werden. Eine besondere Möglichkeit der Einbeziehung von Wirkstoffen ergibt sich beim Lösungsspinnen. Beim Lösungsspinnen wird das vorzugsweise resorbierbare Polymer in einem Lösungsmittel in der Regel bei Raumtemperatur aufgelöst. In diese Lösung kann der Wirkstoff mit eingebracht werden, sofern er mit dem Lösungsmittel verträglich ist. Damit befindet sich der Wirkstoff bereits in dem lösungsgesponnenen Faden, der zu einem Rohr aufgewickelt wird.

Beim anschließenden Vereinigen der Polymerrohre durch Verschmelzen sind die Temperaturen deutlich niedriger und damit wirkstoffschonender als beim Extrudieren oder Spritzgießen. Beim Extrudieren bzw. Spritzgießen der Polymerrohre werden die Schmelztemperaturen der Polymere deutlich überschritten.

Auch kann ein Wirkstoffgradient über die Wandstärke des Polymerrohrs vorteilhaft sein. Hierzu können z.B. Mehrkomponentenfäden mit unterschiedlicher Wirkstoffkonzentration bzw. mit unterschiedlichen Wirkstoffen zur Herstellung des Polymerrohrs verwendet werden. Alternativ können einem kontinuierlichen Mehrkomponentenfaden bei dessen Herstellung unterschiedliche Wirkstoffkonzentrationen bzw. unterschiedliche Wirkstoffe zugesetzt werden. In Kombination mit dem mindestens einen Wirkstoff oder der Wirkstoffkombination können weitere Substanzen z.B. Transportvermittler eingebracht werden.

Als Lösungsmittel eignen sich bevorzugt organische Lösungsmittel wie beispielsweise Chloroform (Trichlormethan), Methylenchlorid (Dichlormethan), Trifluorethanol (TFE), Hexafluoroisopropanol (HFIP), Tetrahydrofuran (THF), Aceton (Dimethylketon), Diethylether, Methanol, Ethanol, Propanol, Isopropanol, Diethylketon, Dimethylformamid (DMF), Dimethylacetamid, Essigsäureethylester, Dimethylsulfoxid (DMSO), Benzol, Toluol, Xylol, t-Butylmethylether (MTBE), Cyclohexan, N-Methyl-2-Pyrrolidone, Pentan, Hexan, Heptan, wobei Methylenchlorid und Chloroform bevorzugt sind.

Die spätere Freisetzung der Wirkstoffe erfolgt mit der Implantation der porenfreien Polymerrohre bzw. der daraus hergestellten Implantate wie Stents. In der Anfangsphase nach der Implantation von Stents, hergestellt aus den erfindungsgemäßen Polymerrohren, finden Zersetzungsvorgänge der Polymermoleküle statt, ohne dass der Stent degradiert, d.h. ohne Masseverluste. Die Wirkstoffabgabe erfolgt im Wesentlichen durch Diffusion. Bei der später einsetzenden Degradation erfolgt die Wirkstoffabgabe durch Erosion. Möglich ist auch, die Wirkstoffe in unterschiedlichen Konzentrationen in die Rohrwand einzubringen und damit dem Verlauf der Diffusion und der Degradation des Stents und/oder dem Heilungsprozess des Gefäßes Rechnung zu tragen. Im Gegensatz dazu wird bei beschichteten, resorbierbaren und nicht resorbierbaren Polymerstents erst die Wirkstoffbeschichtung abgebaut und danach der Stent, ohne dass noch Wirkstoffe vorhanden sind.

Dennoch ist es auch möglich, die erfindungsgemäß hergestellten Polymerrohre bzw. Polymerstents mit einer zusätzlichen Beschichtung zu versehen. Dabei handelt es sich bevorzugt um eine wirkstofffreisetzende Beschichtung. Der Wirkstoff oder eine Wirkstoffkombination kann dabei alleine oder in einer geeigneten Matrix auf die polymere Oberfläche der Rohre oder Stents aufgebracht werden. Eine geeignete Matrix kann ein Polymer, bevorzugt ein resorbierbares Polymer, sein. Das Polymer der wirkstofffreisetzenden Beschichtung kann dabei mit dem Polymer des Grundgerüsts identisch sein.

Mit dem Wirkstoff zusammen können auch weitere Substanzen aufgebracht werden, die dessen Freisetzung bzw. Haftung auf dem Stent beeinflussen. Als pharmakologisch verträgliche Träger in einer wirkstofffreisetzende Beschichtung können Substanzen dienen, ausgewählt aus der Gruppe bestehend aus oder umfassend: Stärke, Natrium-Carboxymethylstärke, Sorbitol, Sucrose, Magnesiumstearat, Dicalciumphosphat, Calciumsulfat, Talk, Mannitol, Polyvinylalkohole, Polyvinylpyrrolidon, Gelatine, natürliche Zucker, sowohl natürliche als auch synthetische Gummis wie beispielsweise Akaziengummi oder Guar-Gummi, Natriumalginat, Natriumbenzoat, Natriumacetat, Glyceride, Isopropylmyristate und - palmitate, Citrate, wie Tributyl- und Triethylcitrate und deren Acetylderivate, Phtalate wie Dimethylphtalat oder Dibutylphtalat, Benzoesäurebenzylester, Triacetin, 2-Pyrrolidon, Agar, Cellulose, Cellulosederivate wie Methylcellulose, NatriumCarboxymethylcellulose und Hydroxypropylmethylcellulose,.

Die wirkstofffreisetzende Beschichtung kann mittels bekannter Verfahren wie beispielsweise Sprühverfahren, Tauchverfahren, Plasmaverfahren, Pinselverfahren, Spritzenverfahren, Elektrospinnen oder Pipettierverfahren auf das Grundgerüst (bestehend aus Stentstreben, die aus dem erfindungsgemäßen Polymerrohr geschnitten wurden) aufgebracht werden.

Aus den obigen Ausführungen wird deutlich, dass die Eigenschaften der erfindungsgemäßen Polymerrohre auch Eigenschaften der aus diesen Rohren hergestellten Stents bzw. Gefäßstützen sind. Somit sollen alle hierin angeführten Merkmale und Merkmalskombinationen der erfindungsgemäßen Polymerrohre auch im Zusammenhang mit den erfindungsgemäßen Stents verstanden werden. Sowohl die Polymerrohre als auch die Stents werden in ihrer Charakteristik vor allem durch die erfindungsgemäßen Herstellungsverfahren geprägt. Die vorliegende Erfindung betrifft demnach auch Polymerrohre und Stents erhältlich nach einem der erfindungsgemäßen Verfahren. Die Polymerrohre und Stents hergestellt durch eins der erfindungsgemäßen Verfahren zeichnen sich durch eine erhöhte radiale Festigkeit bei gleichzeitig hoher oder sogar erhöhter Bruchdehnung aus, wobei die radiale Festigkeit bevorzugt > 80 MPa, weiter bevorzugt > 90 MPa, noch weiter bevorzugt > 100 MPa beträgt und die Bruchdehnung > 30 %, weiter bevorzugt > 40 %, noch weiter bevorzugt > 50 % ist.

### Figurenbeschreibung

- **Figur 1:**: Dargestellt sind Querschnitte zweier bevorzugter Ausführungsformen der Mehrkomponentenfäden. Die Mehrkomponentenfäden können beispielsweise ein Kern-Mantel-Profil a) aufweisen, bei dem die Faser-Komponente (2) den Kern bildet, der von der Matrix-Komponente (1) ummantelt ist. Alternativ kann der Mehrkomponentenfaden ein Inseln-im-See-Profil b) besitzen. Dabei ist die Faser-Komponente (2) in Form von vielen parallel angeordneten Polymerfasern eingebettet in der Matrix-Komponente (2). Mehrkomponentenfäden mit diesen Querschnittsprofilen können z.B. im Schmelzspinnverfahren hergestellt werden.
- **Figur 2:**: Skizze der axialen und radialen Zugprobe zur Bestimmung der Festigkeits- und Verformungskenngrößen, wie Streckgrenze, Zugfestigkeit und Bruchdehnung.
- **Figur 3:**: Skizze der Einspannvorrichtung. Die Zugproben werden über ihre vreiten Enden in der Einspannvorrichtung fixiert.
- **Figur 4:**: Skizze der Zugprüfmaschine

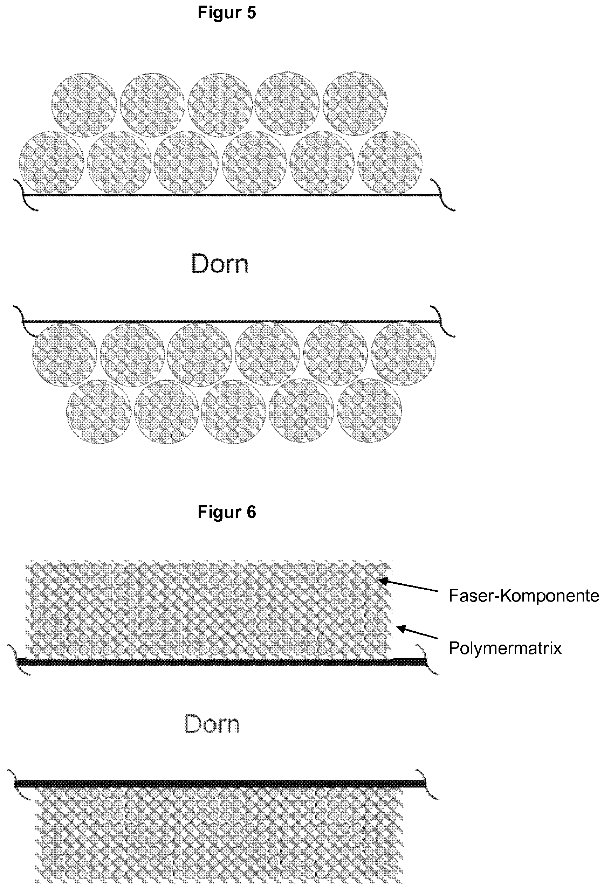

- **Figur 5:**: Skizze aufgewickelter Mehrkomponentenfäden mit einem Inseln-im-See-Profil auf einem Dorn.
- **Figur 6:**: Schematische Darstellung des porenfrei vereinigten Polymerrohrs auf einem Dorn. Durch das schmelzbasierte oder lösungsmittelbasierte Vereinigen haben sich die Poren geschlossen. Die Orientierung der Faser-Komponente bleibt dabei erhalten.
- **Figur 7:**: Entfernung der Rohrstruktur vom Dorn: Durch kraftvolles Herunterschieben wird das porenfreie Polymerrohr vom Dorn entfernt.

### Beispiele

### Beispiel 1: PLLA / PCL Inseln-im-See-Verbundrohr

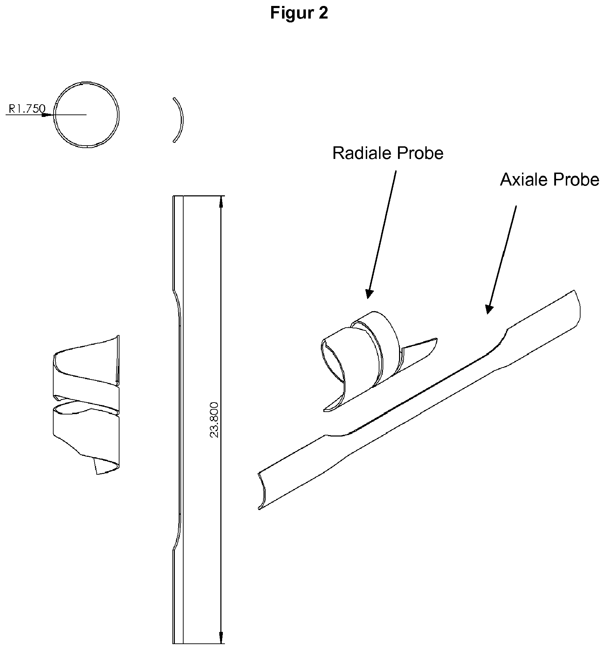

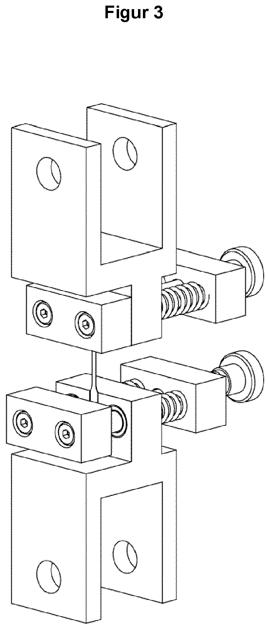

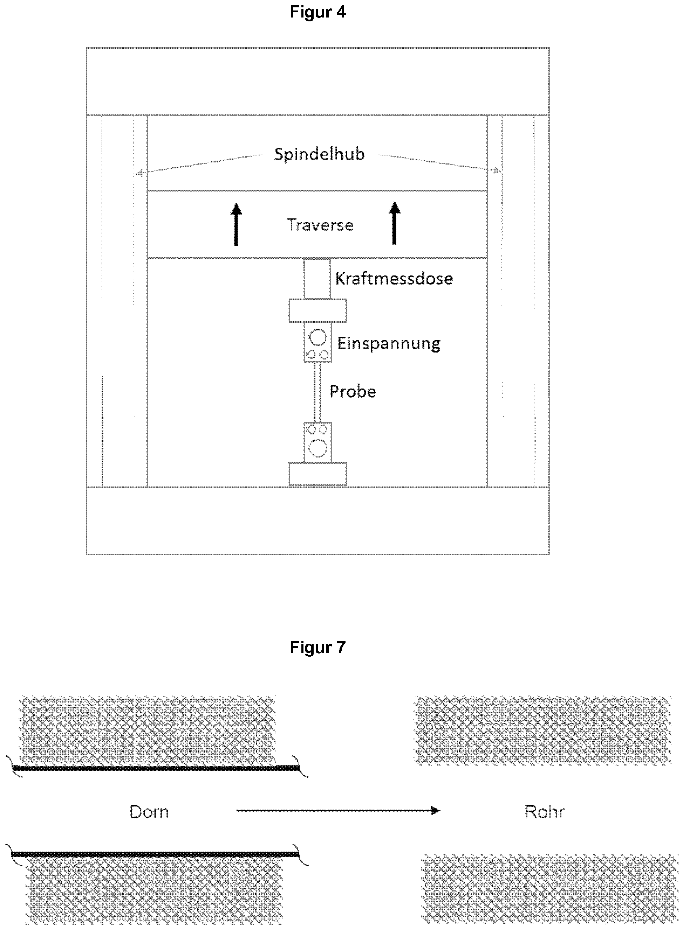

Der Herstellungsprozess umfasst folgende Schritte:
1. Verspinnen von PLLA (Insel) und PCL (Poly(ε-caprolacton)) (See) zu einer Inseln-im-See-Faser im Schmelzspinnverfahren
2. Verstrecken der Faser bis zu einer Zugfestigkeit von 350 MPa
3. Aufwickeln der Faser auf einen zylindrischen Dorn mit einem Durchmesser von 3 mm
4. Verschmelzen des Matrixpolymers (PCL) durch Widerstandserwärmung des Dorns auf 75 °C im Vakuum
5. Abziehen des verschmolzenen Faserverbunds vom Dorn durch kraftvolles Herunterschieben (**Figur 7**)
6. Einspannung des Rohres in die Drehachse einer Ultrakurzpulslaseranlage
7. Laserschneiden des Stents und der Zugproben (**Figur 2**)
8. Einspannen der Zugproben in den Zugprobenhalter (**Figur 3**)
9. Aufzeichnen eines Spannungs-Dehnungs-Diagramms mithilfe einer Zugprüfmaschine (**Figur 4**)

### Beispiel 2: PGA / PHB Kern-Mantel-Verbundrohr

1. Verspinnen von PGA (Kern) und PHB (Mantel) zu einer Kern-Mantel-Faser im Schmelzspinnverfahren
2. Verstrecken der Faser bis zu einer Zugfestigkeit von 500 MPa
3. Aufwickeln der Faser auf einen zylindrischen Dorn mit einem Durchmesser von 4 mm
4. Verschmelzen des Matrixpolymers (PHB) durch Widerstandserwärmung des Dorns auf 175 °C im Vakuum
5. Abziehen des verschmolzenen Faserverbunds vom Dorn durch kraftvolles Herunterschieben (**Figur 7**)
6. Einspannung des Rohres in die Drehachse einer Ultrakurzpulslaseranlage
7. Laserschneiden des Stents und der Zugproben (**Figur 2**)
8. Einspannen der Zugproben in den Zugprobenhalter (**Figur 3**)
9. Aufzeichnen eines Spannungs-Dehnungs-Diagramms mithilfe einer Zugprüfmaschine (**Figur 4**)

### Beispiel 2: PGA / PHB Kern-Mantel-Verbundrohr

1. Verspinnen von PGA (Kern) und PHB (Mantel) zu einer Kern-Mantel-Faser im Schmelzspinnverfahren
2. Verstrecken der Faser bis zu einer Zugfestigkeit von 500 MPa
3. Aufwickeln der Faser auf einen zylindrischen Dorn mit einem Durchmesser von 4 mm
4. Verschmelzen des Matrixpolymers (PHB) durch Widerstandserwärmung des Dorns auf 175 °C im Vakuum
5. Abziehen des verschmolzenen Faserverbunds vom Dorn durch kraftvolles Herunterschieben (**Figur 7**)
6. Einspannung des Rohres in die Drehachse einer Ultrakurzpulslaseranlage
7. Laserschneiden des Stents und der Zugproben (**Figur 2**)
8. Einspannen der Zugproben in den Zugprobenhalter (**Figur 3**)
9. Aufzeichnen eines Spannungs-Dehnungs-Diagramms mithilfe einer Zugprüfmaschine (**Figur 4**)

### Beispiel 3: PGA / PLLA-Faserverbund durch koaxiales Elektrospinnen

1. Auflösen von PGA in HFIP mit einer Konzentration von 5 Gewichtsprozenten.
2. Auflösen von PCL in TFE mit einer Konzentration von 10 Gewichtsprozenten.
3. Koaxiales Verspinnen von PGA (Faser) und PLLA (Matrix) im Elektrospinnverfahren und gleichzeitiges Aufwickeln auf einen schnell drehenden 3,3 mm starken Dorn.
4. Verschmelzen des Matrixpolymers (PLLA) durch Widerstandserwärmung des Dorns auf 190 °C im Vakuum
5. Abziehen des verschmolzenen Faserverbundrohrs durch kraftvolles Herunterschieben
6. Einspannung des Rohres in die Drehachse eines Ultrakurzpulslasers
7. Laserschneiden des Stents und der Zugproben (**Figur 2**)
8. Einspannen der Zugproben in den Zugprobenhalter (**Figur 3**)
9. Aufzeichnen eines Spannungs-Dehnungs-Diagramms mithilfe einer Zugprüfmaschine (**Figur 4**)

### Beispiel 4: PLLA / PCL-Faserverbund durch lösungsmittelbasiertes Vereinigen

Der Herstellungsprozess umfasst folgende Schritte:
1. Verspinnen von PLLA (Insel) und PCL (See) zu einer Inseln-im-See-Faser im Lösungsspinnverfahren
2. Verstrecken der Faser bis zu einer Zugfestigkeit von 420 MPa
3. Aufwickeln der Faser auf einen zylindrischen Dorn mit einem Durchmesser von 2,8 mm
4. Anlösen des Matrixpolymers (PCL) durch Eintauchen des Dorns in das Lösungsmittel TFE. Rotieren des Dornes im Lösungsmittel bis gesamte Struktur angelöst ist.
5. Herausziehen des Domes und Trocknen des Domes in einem Vakuumtrockenofen bei 40 °C.
6. Abziehen des verschmolzenen Faserverbunds vom Dorn durch kraftvolles Herunterschieben (**Figur 7**)
7. Einspannung des Rohres in die Drehachse einer Ultrakurzpulslaseranlage
8. Laserschneiden des Stents und der Zugproben (**Figur 2**)
9. Einspannen der Zugproben in den Zugprobenhalter (**Figur 3**)
10. Aufzeichnen eines Spannungs-Dehnungs-Diagramms mithilfe einer Zugprüfmaschine (**Figur 4**)

## Patentansprüche

1. Polymerrohr bestehend aus mindestens einem gerichtet aufgewickelten Mehrkomponentenfaden mit einem Durchmesser ≤ 200 µm, wobei der mindestens eine gerichtet aufgewickelte Mehrkomponentenfaden aus einer Faser-Komponente und einer Matrix-Komponente besteht.

2. Polymerrohr gemäß Anspruch 1, wobei die Matrix-Komponente unter chemischen und/oder physikalischen Bedingungen plastisch verformbar ist unten denen die Faser-Komponente nicht plastisch verformt wird.

3. Polymerrohr gemäß Anspruch 1 oder 2, wobei die Matrix-Komponente nicht jedoch die Faser-Komponente unter Einwirkung von Trifluorethanol oder Chloroform oder Dichlormethan oder Hexafluorisopropanol oder eines Gemisches aus diesen Lösungsmitteln plastisch verformbar ist.

4. Polymerrohr gemäß Anspruch 1, 2 oder 3, wobei die Faser-Komponente entlang der Längsachse des Mehrkomponentenfadens verläuft und die Matrix-Komponente die Faser-Komponente umgibt.

5. Mehrkomponentenfaden mit einem Durchmesser ≤ 200 µm zur Herstellung des Polymerrohrs gemäß Anspruch 1 bestehend aus einer Faser-Komponente und einer Matrix-Komponente, wobei die Faser-Komponente entlang der Längsachse des Mehrkomponentenfadens verläuft und die Matrix-Komponente die Faser-Komponente umgibt und die Matrix-Komponente unter chemischen und/oder physikalischen Bedingungen plastisch verformbar ist unten denen die Faser-Komponente nicht plastisch verformt wird.

6. Mehrkomponentenfaden gemäß Anspruch 5 bestehend aus einer Materialkombination für die Matrix-Komponente und die Faser-Komponente ausgewählt aus der Gruppe bestehend aus:
Polyglycolid / PLLA, PDLLA / PLLA, PLLA / PLLA-Polyglycolid-Copolymer,
PLLA-Polyglycolid-Copolymer / PLLA-Poly(ε-caprolacton)-Copolymer, PDLLA /
PLLA-Polyglycolid-Copolymer, PLLA / Poly(ε-caprolacton) und
Polyglycolid / Polyhydroxybutyrat.

7. Stent bestehend aus einem lasergeschnittenen Polymerrohr gemäß Anspruch 1.

8. Herstellungsverfahren umfassend den folgenden Schritt: gerichtete Aufwicklung mindestens eines Mehrkomponentenfadens mit einem Durchmesser ≤ 200 µm, bestehend aus einer Faser-Komponente und einer Matrix-Komponente, zu einem Polymerrohr gemäß Anspruch 1.

9. Herstellungsverfahren gemäß Anspruch 8 des Weiteren umfassend den folgenden Schritt: plastische Verformung der Matrix-Komponente unter chemischen und/oder physikalischen Bedingungen unten denen die Faser-Komponente nicht plastisch verformbar ist zwecks Herstellung eines porenfreien Polymerrohrs und laserschneiden eines Stents aus dem porenfreien Polymerrohr.

10. Herstellungsverfahren gemäß Anspruch 9, wobei die plastische Verformung der Matrix-Komponente lösungsmittelbasiert erfolgt oder wobei die plastische Verformung der Matrix-Komponente lösungsmittelbasiert durch Einwirkung von Trifluorethanol oder Chloroform oder Dichlormethan oder Hexafluorisopropanol oder eines Gemisches aus diesen Lösungsmitteln erfolgt.

11. Stent erhältlich durch ein Herstellungsverfahren gemäß einem der Ansprüche 8, 9 oder 10.

12. Stent bestehend aus einem plastisch verformten lasergeschnittenen porenfreien Polymerrohr, wobei das Polymerrohr aus mindestens einem gerichtet aufgewickelten Mehrkomponentenfaden mit einem Durchmesser ≤ 200 µm besteht und der mindestens eine gerichtet aufgewickelte Mehrkomponentenfaden aus einer Faser-Komponente und einer Matrix-Komponente besteht und die Faser-Komponente entlang der Längsachse des mindestens einen Mehrkomponentenfadens verläuft und die Matrix-Komponente die Faser-Komponente umgibt.

13. Stent mit einer radialen Rückstellkraft in Richtung des Durchmessers des Polymerrohrs aus dem der Stent hergestellt wurde.
